(19) Europäisches Patentamt
European Patent Office
Office européen des brevets

(11) Numéro de publication: **0 053 542**
**A1**

(12) **DEMANDE DE BREVET EUROPEEN**

(21) Numéro de dépôt: **81401833.9**

(22) Date de dépôt: **19.11.81**

(51) Int. Cl.³: **C 07 D 501/24**, A 61 K 31/545

(30) Priorité: **20.11.80 FR 8024638**

(43) Date de publication de la demande: **09.06.82**
**Bulletin 82/23**

(84) Etats contractants désignés: **AT BE CH DE FR GB IT LI LU NL SE**

(71) Demandeur: **RHONE-POULENC INDUSTRIES,**
**22 Avenue Montaigne, F-75008 Paris (FR)**

(72) Inventeur: **Berger, Christian, 26 A rue Paul Rivet, F-92350 Le Plessis Robinson (FR)**
Inventeur: **Farge, Daniel, 30 rue des Pins Sylvestres, F-94320 Thiais (FR)**
Inventeur: **Moutonnier, Claude, 3 rue Auguste Rodin, F-92350 Le Plessis Robinson (FR)**
Inventeur: **Wolff, Gérard, 7 rue Jeanne d'Arc, F-94320 Thiais (FR)**

(74) Mandataire: **Herson, Jean et al, RHONE-POULENC RECHERCHES Brevets Pharma 25, quai Paul Doumer, F-92408 Courbevoie Cedex (FR)**

(54) **Nouveaux dérivés de la céphalosporine, leur préparation et les médicaments qui les contiennent.**

(57) Nouveaux dérivés de la céphalosporine de formule générale (I) dans laquelle R est un radical hétérocyclyle substitué ou non, R° représente divers radicaux organiques et R' est un atome d'hydrogène, un radical alcoyle, vinyle, cyanométhyle ou un radical de formule générale (II) dans laquelle $R'_a$ et $R'_b$ sont des atomes d'hydrogène, des radicaux alcoyle ou forment ensemble un radical alcoylène, ainsi que leurs sels, leur préparation et les médicaments qui les contiennent.

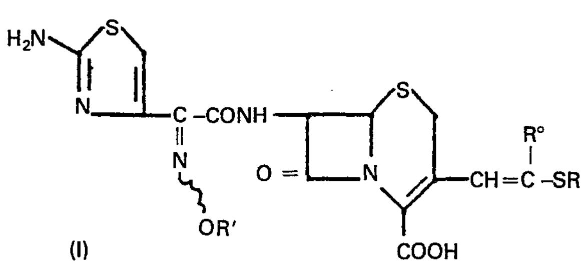

(I)

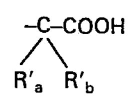

(II)

EP 0 053 542 A1

La présente invention concerne de nouvelles vinyl-3 céphalosporines de formule générale :

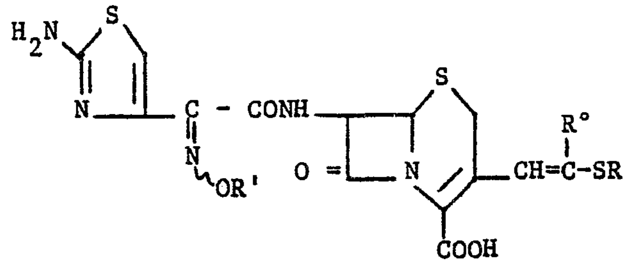

(I)

leurs sels, leur préparation et les médicaments qui les contiennent.

Dans la formule générale (I),

le symbole R est choisi parmi les significations suivantes :

1) pyridyle-2, -3 ou -4 éventuellement N-oxydés
2) pyrimidinyle-2
3) méthyl-6 oxyde-1 pyridazinyle-3
4) dioxo-5,6 tétrahydro-1,4,5,6 triazine-1,2,4 yle-3 substitué en position -4 par
   a) un radical alcoyle contenant 1 ou 2 atomes de carbone éventuellement substitué par un radical alcoyloxy, alcoylthio, formyle,
   b) un radical allyle, dihydroxy-2,3 propyle, dihydroxy-1,3 propyle-2 ou formyl-2 hydroxy-2 éthyle,
   c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,
5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle.
6) alcoyl-2 dihydro-2,5 oxo-5 triazine-1,2,4 yle-3 éventuellement

substitué en position -6 par un radical alcoyle ou alcoyloxy dont les portions et radicaux alcoyles contiennent 1 ou 2 atomes de carbone,

7) amino-1 dihydro-1,2 oxo-2 pyrimidinyle-4

8) thiadiazol-1,3,4 yle-5 substitué par alcoyle, dialcoylaminoalcoyle ou acylaminoalcoyle,

9) tétrazolyle-5 substitué en position -1 par

a) un radical alcoyle contenant 1 ou 2 atomes de carbone éventuellement substitué par un radical formyle,

b) un radical alcoyle contenant 2 ou 3 atomes de carbone substitué par hydroxy, acylamino, ou dialcoylamino, ou

10) a) alcoyl-1 triazol-1,2,4 yle-5 éventuellement substitué en position-3 par un radical alcoyloxycarbonyle dont les radicaux alcoyle et alcoyloxy contiennent 1 ou 2 atomes de carbone

b) alcoyl-1 triazol-1,3,4 yle-5

le symbole R° est un radical d'un premier groupe constitué par un radical phényle éventuellement substitué (par un radical alcoyle, trifluorométhyle, dialcoylaminométhyle, alcoyloxy, alcoylthio, dialcoylamino ou par un atome d'halogène tel que le fluor ou le chlore), un radical hétérocyclyle à 5 ou 6 chaînons contenant un seul hétéroatome (tel que pyridyle-2 ou -3, thiényle-2 ou -3 ou furyle-2 ou -3) éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons, ou un radical hétérocyclyle à 5 ou 6 chaînons contenant un atome d'oxygène ou de soufre (dans ce qui suit, le radical du second groupe sera désigné comme "radical $-CH\ R_9R_{10}$")

et le symbole R' est un atome d'hydrogène, un radical alcoyle, vinyle, cyanométhyle ou un radical de formule générale :

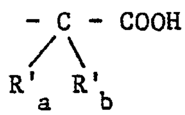

(II)

(dans laquelle $R'_a$ et $R'_b$, qui sont identiques ou différents, sont des atomes d'hydrogène ou des radicaux alcoyle ou forment ensemble un radical alcoylène contenant 2 ou 3 atomes de carbone) étant entendu que, sauf mention spéciale, les portions ou radicaux alcoyles ou acyles cités ci-dessus (ou qui seront cités ci-après) sont droits ou ramifiés et contiennent 1 à 4 atomes de carbone.

Il est également entendu que les substituants R° et -SR peuvent être situés en position cis ou trans par rapport au noyau céphalosporine et que ces isomères et leurs mélanges entrent dans le cadre de la présente invention.

Dans ce qui suit la stéréoisomérie trans sera désignée par E et la stéréoisomérie cis sera désignée par Z.

Par ailleurs, il est entendu que le groupement -OR' peut se trouver dans l'une des positions syn ou anti et que ces isomères et leurs mélanges entrent aussi dans le cadre de la présente invention.

La forme syn peut être représentée par la formule :

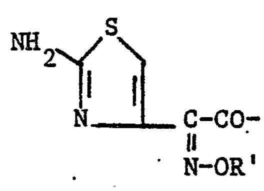

(IIIa)

La forme anti peut être représentée par la formule :

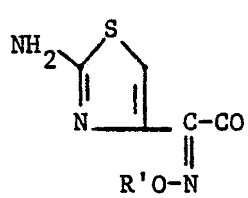

(IIIb)

Lorsque le radical R est un radical tétrahydro-1,4,5,6 triazinyle substitué en position -1 ou -4 ou tétrahydro-1,2,5,6 triazinyle substitué en position -2, il peut être représenté par les formes tautomères :

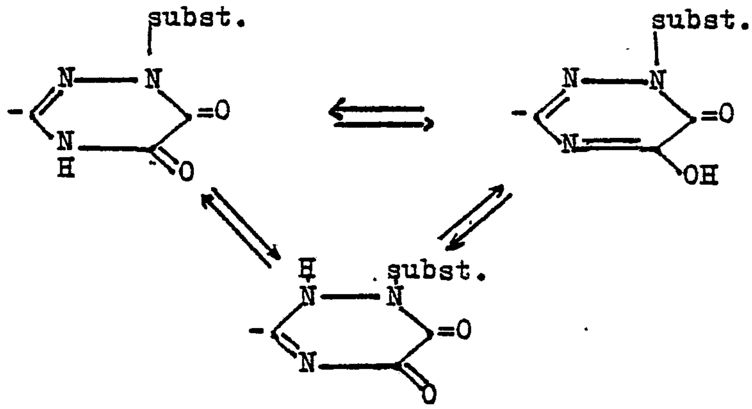

(IVa)

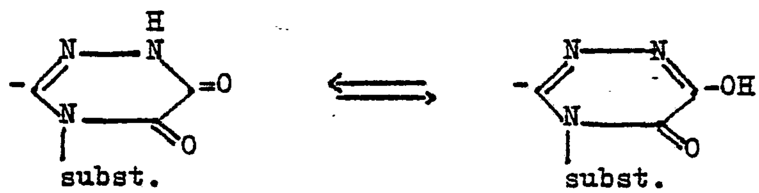

(IVb)

ou

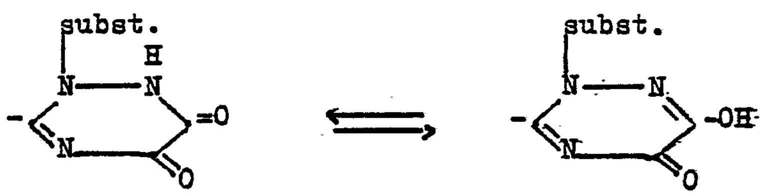

(IVc)

Lorsque le radical R contient un substituant formylalcoyle, il peut se présenter sous sa forme aldéhyde libre ou hydrate d'aldéhyde. On observe notamment ces formes dans les conditions décrites ci-dessous :

les études de résonance magnétique nucléaire montrent par exemple que lorsque R est dioxo-5,6 formylméthyl-4 tétrahydro-1,4,5,6 triazine-1,2,4 yle-3 :

- en solvant acide, tel que l'acide formique ou trifluoroacétique (deutérés), en présence ou non d'eau (lourde) le produit se présente principalement sous la forme d'aldéhyde libre.
- en solvant basique tel que l'eau (lourde) additionnée de bicarbonate de sodium, il se présente principalement sous la forme d'hydrate d'aldéhyde.

- en solvant neutre tel que diméthylsulfoxyde ($d_6$), les formes aldéhyde libre et hydrate d'aldéhyde sont présentes, l'addition d'eau déplaçant l'équilibre en faveur de la forme hydrate d'aldéhyde.

I-/ Selon l'invention, les thiovinyl-3 céphalosporines de formule générale (I) peuvent être préparées par action d'un thiol (ou d'un de ses sels alcalins ou alcalino-terreux) de formule générale :

$$R-SH \qquad (V)$$

dans laquelle R, qui est défini comme précédemment, est éventuellement protégé, sur un dérivé de la céphalosporine (ou un mélange des isomères) de formule générale :

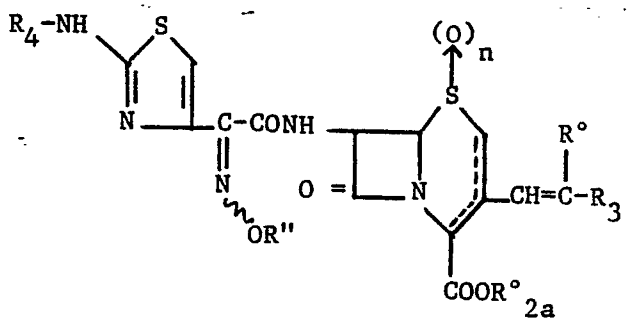

(VI)

[dans laquelle R° est défini comme précédemment, R" est défini comme R' précédemment ou représente un radical protecteur d'oxime tel que trityle, tétrahydropyrannyle ou méthoxy-2 propyle-2 ou un radical de formule générale (II) dans laquelle le groupement carboxy est protégé par un radical protecteur d'acide, par exemple tel que défini pour $R°_{2a}$, $R_4$ est un atome d'hydrogène ou un radical protecteur d'amine tel que t.butoxycarbonyle, trityle, benzyle, dibenzyle, benzyloxycarbonyle, p.nitrobenzyloxycarbonyle ou p.méthoxybenzyloxycarbonyle ou tel qu'un radical phosphoré défini ci-après par la formule générale (XVII),

$R^{\circ}_{2a}$ représente un atome d'hydrogène ou un radical protecteur d'acide tel que méthoxyméthyle, t.butyle, benzhydryle, benzyle, nitrobenzyle ou p.méthoxybenzyle,

$R_3$ représente un radical de formule générale :

$$R'_3\ SO_2O- \qquad (VII)$$

dans laquelle $R'_3$ représente un radical alcoyle, trihalogénométhyle (par exemple trifluorométhyle) ou phényle éventuellement substitué par un atome d'halogène ou par un ou plusieurs radicaux alcoyle ou nitro, et

n égale 0 ou 1

étant entendu que lorsque n = 0 le produit se présente sous forme bicyclooctène-2 et/ou -3 (selon la nomenclature des Chemical Abstracts) et lorsque n = 1 le produit se présente sous forme bicyclooctène-2) suivie de la réduction du sulfoxyde obtenu lorsque n = 1 et le cas échéant de l'élimination des radicaux protecteurs.

Lorsque l'on veut obtenir un produit de formule générale (I) dans laquelle R contient un radical formyle, on met en oeuvre un thiol de formule générale (V) dans laquelle ce radical est protégé à l'état d'acétal de formule générale :

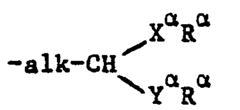
(VIIIa)

ou

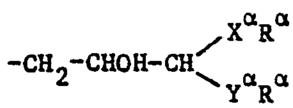
(VIIIb)

dans lesquelles alk est un radical alcoylène contenant 1 ou 2 atomes de carbone, $X^{\alpha}$ et $Y^{\alpha}$ sont identiques et représentent des atomes d'oxygène ou de soufre et $R^{\alpha}$ représente un radical alcoyle, ou bien $X^{\alpha}$ et $Y^{\alpha}$ sont identiques ou différents et représentent des atomes d'oxygène ou de soufre et les radicaux $R^{\alpha}$ forment ensemble un radical alcoylène contenant 2 ou 3 atomes de carbone.

Il est entendu que, lorsque le radical R du thiol R-SH (V) contient un groupement susceptible d'interférer avec la réaction, il est préférable de protéger ce groupement, par toute méthode connue en soi et qui n'altère pas le reste de la molécule.

S'il s'agit d'un groupement amino ou alcoylamino, la protection s'effectue par un radical protecteur tel que $R_4$ défini précédemment.

S'il s'agit de groupements hydroxy la protection s'effectue par des radicaux trityle, tétrahydropyrannyle ou méthoxy-2 propyle-2 ou bien, lorsqu'il s'agit d'un radical dihydroxy-2,3 propyle ou dihydroxy-1,3 propyle-2, à l'état d'acétal cyclique sous forme d'un radical diméthyl-2,2 dioxolannyl-4 méthyle ou diméthyl-2,2 dioxannyle-5.

Par ailleurs il est entendu que, lorsque le radical R du produit de formule générale (V) contient un radical hydroxy ou sulfonyle, il est préférable de mettre en oeuvre un produit de formule générale (VI) dans laquelle n = 0.

Il est également entendu que, lorsque dans la formule générale (VI) R" représente un atome d'hydrogène, il est préférable de protéger l'oxime par un radical protecteur tel que décrit précédemment.

La réaction des produits de formules générales (V) et (VI) s'effectue généralement en présence d'une base telle qu'une pyridine ou une base organique tertiaire de formule générale

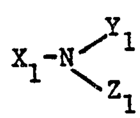

(IX)

dans laquelle $X_1$, $Y_1$ et $Z_1$ représentent des radicaux alcoyle ou phényle, ou éventuellement deux d'entre eux forment un cycle avec l'atome d'azote auquel ils sont rattachés. On utilise par exemple la diisopropyléthylamine ou la diéthylphénylamine.

Lorsque l'on utilise un sel du thiol de formule générale (V) il n'est pas nécessaire d'opérer en présence d'une base organique telle que définie ci-dessus.

On opère avantageusement dans un solvant organique tel que le diméthylformamide, le tétrahydrofuranne, l'acétonitrile ou un mélange de tels solvants.

Il est également possible d'opérer en présence de bicarbonate alcalin dans un solvant tel que cité ci-dessus, éventuellement en présence d'eau. On opère à une température comprise entre -20°C et la température de reflux du mélange réactionnel, la température choisie étant variable selon le thiol employé. De même, selon le thiol employé, la durée de réaction peut varier de 5 minutes à 48 heures.

Eventuellement on opère sous azote.

Le cas échéant la réduction du S-oxyde s'effectue selon les méthodes décrites dans la demande de brevet allemand 2 637 176.

L'élimination des radicaux protecteurs peut s'effectuer, à titre d'exemple :

1) pour les groupements protecteurs d'amines :

- lorsqu'il s'agit d'un radical t.butoxycarbonyle, trityle ou p.méthoxybenzyloxycarbonyle : par traitement en milieu acide. De préférence on utilise l'acide trifluoroacétique en opérant à une température comprise entre 0 et 20°C, ou bien on utilise l'acide formique, phosphorique ou polyphosphorique purs ou en présence d'eau, à une température comprise entre 20 et 60°C ou encore l'acide paratoluènesulfonique ou méthanesulfonique dans l'acétone ou l'acétonitrile à une température comprise entre 20°C et la température de reflux du mélange réactionnel.

Dans ces conditions le produit de formule générale (I) peut être obtenu sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique ;

- lorsqu'il s'agit d'un radical benzyle, dibenzyle ou benzyloxycarbonyle : par hydrogénation catalytique,

- lorsqu'il s'agit d'un radical p.nitrobenzyloxycarbonyle : par

réduction, notamment par traitement par le zinc dans l'acide acétique ou par hydrogénolyse,

- lorsqu'il s'agit d'un radical de formule générale (XVII): selon les méthodes citées ci-après pour la préparation des produits de formule générale (XIV),

2) pour les radicaux protecteurs de carboxy :

- lorsqu'il s'agit d'un groupement t.butyle, p.méthoxybenzyle ou benzhydryle : par traitement en milieu acide, dans les conditions décrites ci-avant pour l'élimination du radical trityle protecteur d'amino. Dans le cas du radical benzhydryle, on peut opérer en présence d'anisole

- lorsqu'il s'agit d'un groupement méthoxyméthyle : par traitement en milieu acide dilué

- lorsqu'il s'agit d'un groupement nitrobenzyle : par réduction (notamment traitement par le zinc dans l'acide acétique ou hydrogénolyse).

3) pour les radicaux protecteurs d'oxime :

- lorsqu'il s'agit du radical trityle ou tétrahydropyrannyle : par acidolyse par exemple par l'acide trifluoracétique, l'acide formique aqueux ou non ou l'acide paratoluènesulfonique

- lorsqu'il s'agit du radical méthoxy-2 propyle-2 : selon la méthode décrite dans le brevet belge 875 379.

L'élimination des radicaux protecteurs de groupements hydroxy s'effectue dans les conditions décrites précédemment pour les radicaux protecteurs de l'oxime, c'est-à-dire :

- par acidolyse par exemple par l'acide trifluoracétique, l'acide formique aqueux ou non ou l'acide paratoluènesulfonique lorsqu'il s'agit du radical trityle, tétrahydropyrannyle, diméthyl-2,2 dioxolannyl-4 méthyle ou diméthyl-2,2 dioxannyle-5. Lorsqu'on utilise l'acide formique, aqueux ou non, la libération des radicaux hydroxy protégés à l'état d'acétal cyclique peut conduire au moins partiellement aux mono ou diesters formiques correspondants, qui peuvent être séparés le cas échéant par chromatographie.

- selon la méthode décrite dans le brevet belge 875 379 lorsqu'il s'agit du radical méthoxy-2 propyle-2.

L'élimination des radicaux protecteurs dans les groupements de formule générale(VIIIa)ou(VIIIb)(lorsque l'on veut obtenir un produit de formule générale (I) dans laquelle R contient un radical formyle) s'effectue :

- en présence d'un acide sulfonique (acide méthanesulfonique ou acide p.toluènesulfonique par exemple) dans un solvant organique (acétonitrile ou acétone par exemple), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel.

- ou bien, lorsque le radical R est un radical dioxo-5,6 tétrahydro-1,4,5,6 triazine-1,2,4 yle-3, par action d'acide formique pur ou aqueux (contenant de préférence moins de 10 % d'eau), soit en présence ou non de silice, soit par transacétalisation en présence d'un réactif acétalisable tel que défini ci-dessus.

Il est souvent préférable d'opérer au moyen du sulfoxyde de la céphalosporine de formule générale (VI). Dans le cas contraire, lorsque l'on obtient un mélange d'isomères bicyclooctène-2 et bicyclooctène-3 de formule générale (I), ce mélange peut être oxydé pour obtenir l'oxyde du produit correspondant de formule générale (I) qui se présente sous forme bicyclooctène-2. Cet oxyde peut alors être réduit pour obtenir l'isomère bicyclooctène-2 de formule générale (I).

II-/ Selon l'invention, les thiovinyl-3 céphalosporines de formule générale (I) peuvent également être préparées par action d'un acide de formule générale :

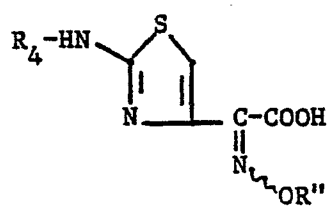

(X)

dans laquelle R" et $R_4$ sont définis comme précédemment, à l'exception de représenter des atomes d'hydrogène (et, pour R", de contenir un radical carboxy libre), ou d'un dérivé de cet acide, sur une amino-7 céphalosporine de formule générale :

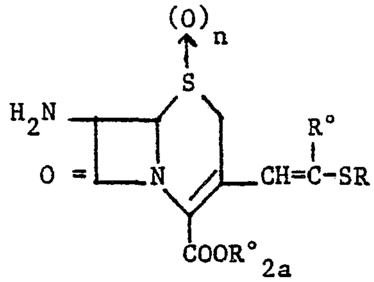

(XI)

dans laquelle R°, R, $R°_{2a}$ et n sont définis comme précédemment, suivie de la réduction de l'oxyde obtenu (lorsque n = 1) et de l'élimination des radicaux protecteurs.

Il est entendu que :

- les radicaux amino ou alcoylamino, qui existent dans certains radicaux R, doivent être protégés, et
- les radicaux carboxy, hydroxy ou formyle contenus dans les radicaux R peuvent être protégés.

La protection et l'élimination des radicaux protecteurs ainsi que la réduction de l'oxyde s'effectuent dans les conditions décrites précédemment.

Lorsque R contient un substituant hydroxy ou sulfonyle, on préfère mettre en oeuvre un produit de formule générale (XI) dans laquelle n = 0.

Il est également entendu que l'acide de formule générale (X) sous forme syn, anti ou leurs mélanges, conduit respectivement aux produits de formule générale (I) de forme syn, anti ou leurs mélanges.

Généralement on effectue la condensation du produit de formule générale (X), dont la fonction acide est libre, sur l'amino-7 céphalosporine de formule générale (XI) dans un solvant organique tel que le diméthylformamide, l'acétonitrile, le tétrahydrofuranne, le chlorure de méthylène ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre -20 et 40°C.

Lorsque l'on utilise un dérivé réactif de l'acide de formule générale (X), il est possible de mettre en oeuvre l'anhydride, un anhydride mixte ou un ester réactif de formule générale :

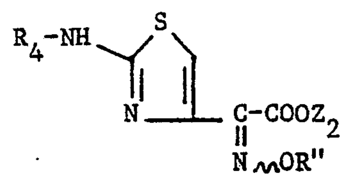

(XII)

[dans laquelle, R" et $R_4$ étant définis comme précédemment, $Z_2$ représente un radical succinimido, benzotriazolyle-1, nitro-4 phényle, dinitro-2,4 phényle, pentachlorophényle ou phtalimido] ou bien un halogénure d'acide, par exemple le chlorure d'acide.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple l'acétone), ainsi que des mélanges de tels solvants [en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine)] ou dans un milieu hydroorganique en présence d'un agent alcalin de condensation tel que le bicarbonate de sodium, et l'on opère à une température comprise entre -40 et +40°C.

Lorsque l'on met en oeuvre un ester réactif de formule générale (XII) on opère généralement en présence d'une trialcoylamine (par exemple triéthylamine) dans un solvant organique tel que le diméthylformamide, à une température comprise entre 0 et 40°C.

Les thiols de formule générale (V), qui peuvent être mis en oeuvre sous leur forme tautomère, peuvent être préparés par application de l'une des méthodes suivantes selon la signification du radical R :

- lorsque R est un radical pyridyle-3 : selon la méthode décrite par H.M. WUEST et E.H. SAKAL, J. Am. Chem. Soc., 73, 1210 (1951),
- lorsque R est un radical oxyde-1 pyridyle-3 : selon la méthode décrite par B. BLANK et coll., J. Med. Chem. 17, 1065 (1974),
- lorsque R est un radical oxyde-1 pyridyle-4 : selon la méthode décrite par R.A.Y. JONES et coll., J. Chem. Soc. 2937 (1960),
- lorsque R est un radical méthyl-6 oxyde-1 pyridazinyle-3 : selon la méthode décrite dans le brevet belge 787 635,
- lorsque R est

1°/- un radical dioxo-5,6 tétrahydro-1,4,5,6 triazine-1,2,4 yle-3 substitué en position -4 par un radical $R^{\gamma}$ choisi parmi :

a) un radical allyle, alcoyle (1 ou 2 atomes de carbone, lui-même éventuellement substitué par un radical alcoyloxy ou alcoylthio),

b) un radical dihydroxy-2,3 propyle ou dihydroxy-1,3 propyle-2 (éventuellement protégés sous forme d'acétal cyclique)

c) un radical alcoyle [2 à 3 atomes de carbone, lui-même substitué par hydroxy, carbamoyloxy, alcoylsulfonylamino, acylamino (éventuellement substitué), uréido, alcoyluréido ou dialcoyluréido],

d) un radical de formule générale (VIIIa) ou (VIIIb),

2°/- un radical dioxo-5,6 tétrahydro-1,4,5,6 triazine-1,2,4 yle-3 substitué en position -1, ou dioxo-5,6 tétrahydro-1,2,5,6 triazine-1,2,4 yle-3 substitué en position -2 par un radical alcoyle contenant 1 ou 2 atomes de carbone ou par un radical de formule générale (VIIIa) : en faisant agir un oxalate d'alcoyle sur une thiosemicarbazide de formules générales :

$$R^{\gamma}\ NH\ CS\ NH{-}NH_2 \qquad (XIIIa)$$

$$H_2NCS\ NH\ NH{-}R^{\gamma} \qquad (XIIIb)$$

$$H_2N\ CS\ \underset{\displaystyle R^{\gamma'}}{\underset{|}{N}}{-}NH_2 \qquad (XIIIc)$$

dans lesquelles $R^{Y}$ a la définition donnée ci-dessus en 1°/- et $R^{Y'}$ est un substituant défini ci-dessus en 2°/-, en présence d'un alcoolate alcalin, par exemple l'éthylate ou le méthylate de sodium ou le t.butylate de potassium, par application de la méthode décrite par M. PESSON et M. ANTOINE, Bull. Soc. Chim. France 1590 (1970).

Il n'est pas absolument nécessaire de purifier le produit obtenu (ni de libérer les radicaux protégés) pour le mettre en oeuvre pour la préparation des produits de formule générale (I).

La thiosemicarbazide de formule générale (XIIIa), (XIIIb) ou (XIIIc) peut être préparée selon l'une des méthodes décrites par K.A. JENSEN et coll., Acta Chem. Scand., 22, 1 (1968), ou par application de la méthode décrite par Y. KAZAKOV et J.Y. POTOVSKII, Doklady Acad. Nauk. SSSR 134, 824 (1960) étant entendu que, lorsque $R^{Y}$ contient un radical amino, ce dernier est protégé.

La protection du radical amino et l'élimination du radical protecteur s'effectuent selon les méthodes habituelles qui n'altèrent pas le reste de la molécule. On utilise notamment le groupement t.butoxycarbonyle, qui peut être éliminé par hydrolyse acide.

- Lorsque R est un radical alcoyl-1 triazol-1,3,4 yle-5 : par application de l'une des méthodes décrites par M. PESSON et M. ANTOINE, Bull. Soc. Chim. France 1590 (1970) ;
- Lorsque R est un radical dioxo-5,6 tétrahydro-1,4,5,6 triazine-1,2,4 yle-3 substitué en position -4 par acyloxyalcoyle (éventuellement substitué) : par acylation de la dioxo-5,6 hydroxyalcoyl-4 mercapto-3 tétrahydro-1,4,5,6 triazine-1,2,4, dont le radical mercapto a été préalablement protégé [par exemple selon C.G. KRUSE et coll., Tet. Lett. 1725 (1976)], par toute méthode connue pour acyler un alcool sans toucher au reste de la molécule, puis libération du groupement mercapto en milieu acide.
- Lorsque R est un radical alcoyl-2 dioxo-5,6 tétrahydro-1,2,5,6 triazine-1,2,4 yle-3, alcoyl-1 triazol-1,2,4 yle-5 ou alcoyl-1 alcoyloxycarbonyl-3 triazol-1,2,4 yle-5 : selon la méthode décrite par M. PESSON et M. ANTOINE, C.R. Acad. Sci., Ser. C, 267, 25, 1726 (1968).

- Lorsque R est un radical alcoyl-2 dihydro-2,5 oxo-5 triazine-1,2,4 yle-3 substitué en position -6 par un radical alcoyle ou alcoyloxy : selon la méthode décrite dans J. Antibiotics, 33, 783 (1980).

- Lorsque R est un radical amino-1 dihydro-1,2 oxo-2 pyrimidinyle-4 : selon la méthode décrite dans la demande de brevet européen EP 00005.

- Lorsque R est un radical thiadiazol-1,3,4 yle-5 éventuellement substitué par alcoyle : selon les méthodes décrites dans le brevet belge 830 821,

- Lorsque R est un radical thiadiazol-1,3,4 yle-5 substitué par dialcoylaminoalcoyle : selon la méthode décrite dans la demande de brevet allemand 2 446 254.

- Lorsque R est un radical thiadiazol-1,3,4 yle-5 substitué par un radical acylaminoalcoyle : selon la méthode décrite dans la demande de brevet japonais 76 80857,

- Lorsque R est un radical tétrazolyle-5 éventuellement substitué en position -1 par alcoyle ou hydroxyalcoyle : selon les méthodes décrites dans le brevet belge 830 821.

- Lorsque R est un radical tétrazolyle-5 substitué en position -1 par un radical dialcoylaminoalcoyle : par application de la méthode décrite dans la demande de brevet allemand 2 738 711.

- Lorsque R est un radical tétrazolyle-5 substitué par un radical acylaminoalcoyle : selon la méthode décrite dans le brevet US 4 117 123.

- Lorsque R est un radical tétrazolyle-5 substitué en position -1 par un radical de formule générale (VIIIa) : par action d'azoture de sodium sur l'isothiocyanate correspondant, par analogie avec la méthode décrite par R.E. ORTH, J. Pharm. Sci. 52 (9), 909 (1963).

Les produits de formule générale (VI) peuvent être préparés par acylation d'une amino-7 céphalosporine de formule générale :

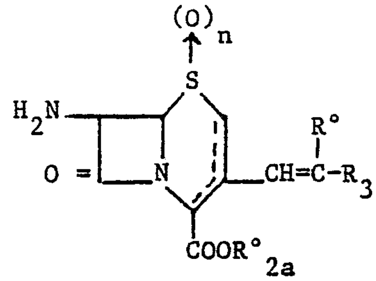

(XIV)

[dans laquelle R°, $R°_{2a}$, $R_3$ et n sont définis comme précédemment et la position de la double liaison ainsi que la configuration du substituant en position -3 est la même que pour le produit de formule générale (VI)] (ou le cas échéant d'un mélange de ses isomères), au moyen d'un acide de formule générale (X) dans laquelle R" et $R_4$ sont définis comme précédemment, ou par action d'un dérivé réactif de cet acide, puis éventuellement réduction du sulfoxyde obtenu et éventuellement élimination des radicaux protecteurs.

On opère dans les conditions décrites précédemment pour la préparation d'une céphalosporine de formule générale (I), les conditions de blocage étant les mêmes.

Le cas échéant la réduction du sulfoxyde et l'élimination des radicaux protecteurs d'acide et/ou d'amine, s'effectuent dans les conditions décrites précédemment.

L'amino-7 céphalosporine de formule générale (XIV) peut être obtenue par élimination du radical $R°_{1a}$ ou éventuellement élimination simultanée des radicaux protecteurs $R°_{1a}$ et $R°_{2a}$ d'un produit de formule générale :

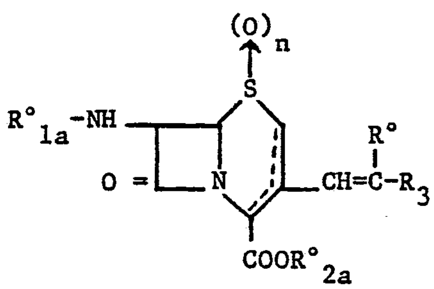

(XV)

dans laquelle R°, $R°_{2a}$, $R_3$ et n étant définis comme précédemment, $R°_{1a}$ représente

un radical benzhydryle, trityle,
un radical acyle de formule générale :

$R_5$-CO- (XVIa)

[[dans laquelle $R_5$ est un radical alcoyle (substitué par un radical phényle ou phénoxy)]]
un radical de formule générale :

$R_6$O CO- (XVIb)

[[dans laquelle $R_6$ est un radical alcoyle ramifié non substitué ou alcoyle ramifié ou droit portant 1 ou plusieurs substituants [choisis parmi phényle et phényle substitué (par un radical alcoyloxy, nitro ou phényle)], ou vinyle]]
un radical de formule générale

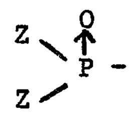

(XVII)

dans laquelle les symboles Z représentent des radicaux phényle ou -OZ' dans lequel Z' représente un radical alcoyle, trichloro-2,2,2 éthyle, phényle ou benzyle (ces deux derniers radicaux pouvant être substitués par un atome d'halogène ou un radical alcoyle, alcoyloxy ou nitro), ou bien les symboles Z' des 2 substituants Z forment ensemble un radical alcoylène contenant 2 ou 3 atomes de carbone
ou bien $R°_{1a}$NH est remplacé par un radical méthylène-amino dans lequel le radical méthylène est substitué par un groupement dialcoylamino ou aryle (lui-même éventuellement substitué par un ou plusieurs radicaux méthoxy ou nitro).

Comme exemples de radicaux $R°_{1a}$ peuvent être cités :

**trityle , phénylacétyle**
**phénoxyacétyle**
**t.butoxycarbonyle**
**benzyloxycarbonyle**
**p.méthoxybenzyloxycarbonyle**
**p.nitrobenzyloxycarbonyle**
**diphénylméthoxycarbonyle**
**(biphénylyl-4)-2 isopropyloxycarbonyle**
**vinyloxycarbonyle**

diméthoxyphosphoryle,
diéthoxyphosphoryle,
diphénoxyphosphoryle,
dibenzyloxyphosphoryle.

Comme exemples de radicaux méthylèneamino on peut citer :
diméthylaminométhylèneamino,
diméthoxy-3,4 benzylidèneamino,
nitro-4 benzylidèneamino.

L'élimination des radicaux protecteurs s'effectue par toute méthode connue pour la libération d'une fonction amine et/ou acide qui n'altère pas le reste de la molécule.

A titre d'exemple :

L'élimination des groupements protecteurs d'amines s'effectue :

- lorsque $R°_{1a}$ représente phénylacétyle ou phénoxyacétyle : selon la méthode décrite dans le brevet belge 758 800,
- lorsque $R°_{1a}$ représente diphénylméthoxycarbonyle, (biphénylyl-4)-2 isopropyloxycarbonyle ou vinyloxycarbonyle et lorsque $R°_{1a}NH$- est remplacé par diméthylaminométhylèneamino, diméthoxy-3,4 benzylidèneamino ou nitro-4 benzylidèneamino : par hydrolyse en milieu acide,
- lorsque $R°_{1a}$ représente un radical trityle, t.butoxycarbonyle, benzyloxycarbonyle, p.méthoxycarbonyle ou p.nitrobenzyloxycarbonyle : selon les méthodes décrites précédemment.
- lorsque $R°_{1a}$ est un radical de formule générale (XVII) : selon la méthode décrite dans le brevet belge 833 619 ou,
- lorsque $R°_{1a}$ est un radical de formule générale (XVII) dans laquelle Z est trichloro-2,2,2 éthyle ou nitrobenzyle : par réduction (notamment traitement par le zinc dans l'acide acétique) ou
- lorsque $R°_{1a}$ est un radical diphénylphosphinoyle : selon la méthode décrite par P. HAAKE et coll., J. Am. Chem. Soc., 95, 8073 (1973).

Les céphalosporines de formule générale (VI) et (XV) peuvent être préparées par action d'une forme activée d'un acide $R'_3SO_3H$ du type :

$(R'_3SO_2)_2O$ (XVIIIa)

$R'_3SO_2Hal$ (XVIIIb)

[$R'_3$ étant défini comme dans la formule (VII) et Hal étant un atome d'halogène] sur une cétone de formule générale :

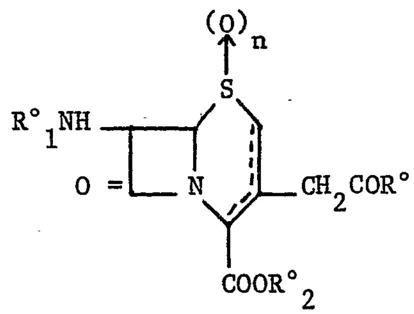

(XIX)

[dans laquelle R° et n étant définis comme précédemment, $R°_1$ soit représente un radical de formule générale

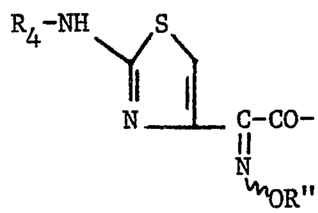

(Xa)

dans laquelle R" et $R_4$ sont définis comme précédemment dans la formule (VI) à l'exception pour $R_4$ de représenter un atome d'hydrogène, soit est défini comme $R°_{1a}$ dans la formule générale (XV), et $R°_2$ est défini comme $R°_{2a}$, et qui se présente sous forme (oxo-2 éthyl)-3 bicyclooctène-2 ou -3 lorsque n = 0 et sous forme (oxo-2 éthyl)-3 bicyclooctène-2 lorsque n = 1] ou sur un mélange de ses isomères, suivie éventuellement de la réduction du sulfoxyde obtenu lorsque n = 1 et éventuellement de l'élimination des radicaux protecteurs de la fonction amine du radical de formule générale (Xa) et/ou des fonctions acides.

Il est entendu que, lorsque dans le radical de formule générale (Xa) R" représente un atome d'hydrogène, il est nécessaire que l'oxime soit protégée. La protection et la libération s'effectuent selon les méthodes décrites précédemment.

On opère généralement en présence

- d'une base tertiaire de formule générale (IX) (par exemple la triéthylamine ou la diméthylaniline)
- ou d'une pyridine substituée par des radicaux alcoyle ou dialcoylamino (par exemple la di-t.butyl-2,6 méthyl-4 pyridine)

- ou d'un amidure de lithium obtenu par réaction d'un alcoyllithium (par exemple le n.butyllithium) sur une amine secondaire de formule générale :

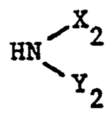

(XX)

dans laquelle $X_2$ et $Y_2$ représentent des radicaux alcoyle ou phényle, ou éventuellement $X_2$ et $Y_2$ forment un cycle avec l'atome d'azote auquel ils sont rattachés (par exemple la diisopropylamine ou la tétraméthyl-2,2,6,6 pipéridine)
- ou d'un alcoolate de métal alcalin (par exemple le t.butylate de potassium)
- ou d'un hydrure de métal alcalin (par exemple l'hydrure de potassium)
- ou d'un alcoyllithium ou d'un aryllithium (par exemple le n-butyllithium ou le mésityllithium),

dans un solvant organique tel qu'un éther (par exemple le dioxanne, le tétrahydrofuranne ou le diméthoxy-1,2 éthane) ou dans un mélange de solvants organiques comprenant un éther (tel que cité ci-dessus) et un solvant tel que l'hexaméthylphosphorotriamide, la N-méthylpyrrolidone ou la diméthyl-1,3 imidazolidinone-2, à une température comprise entre -78°C et la température de reflux du mélange réactionnel éventuellement sous atmosphère inerte (azote ou argon).

La réduction de l'oxyde et l'élimination des groupements protecteurs d'amine, d'acide ou de l'oxime s'effectuent selon les méthodes décrites précédemment.

Les produits de formule générale (XIX) pour lesquels n = 0 peuvent être obtenus par hydrolyse en milieu acide ou neutre d'une énamine de formule générale :

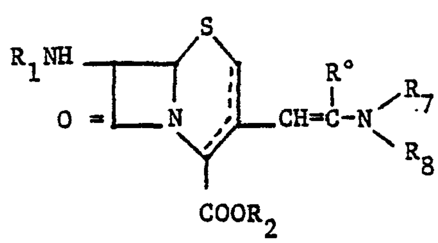

(XXI)

qui se présente sous forme bicyclooctène-2 ou -3 et dans laquelle les substituants R° et $-NR_7R_8$ de la chaîne en position -3 peuvent se trouver en position cis ou trans du bicyclooctène et

$R_1$ représente soit

a) un radical de formule générale (Xa) dans laquelle R" est défini comme dans la formule générale (Xa) à l'exception de contenir un radical carboxy libre et $R_4$ est un radical protecteur tel que défini précédemment, soit

b) un radical tel que défini précédemment pour $R°_{1a}$ dans la formule générale (XV),

$R_2$ représente un radical protecteur tel que défini précédemment pour $R°_{2a}$, R° est défini comme précédemment et

les symboles $R_7$ et $R_8$, qui sont identiques ou différents, représentent des radicaux alcoyle (éventuellement substitués par un radical alcoyloxy ou dialcoylamino) ou phényle ou forment ensemble avec l'atome d'azote auquel ils sont rattachés un hétérocycle saturé à 5 ou 6 chaînons contenant éventuellement un autre hétéroatome choisi parmi l'azote, l'oxygène ou le soufre et éventuellement substitué par un radical alcoyle $\mathcal{J}$, ou d'un mélange de ses isomères, puis élimination éventuelle des radicaux protecteurs d'acide représenté par $R_2$ et/ou contenu par R" (pour obtenir un produit pour lequel $R^°_2$ est un atome d'hydrogène et/ou $R°_1$ contient un groupement carboxy libre).

On opère de préférence dans un acide organique (par exemple acide formique, acide acétique) ou minéral (par exemple acide chlorhydrique, acide sulfurique) en présence ou non d'un solvant, en milieu aqueux ou organique, à une température comprise entre -20°C et la température de reflux du mélange réactionnel, puis on traite éventuellement par une base minérale (bicarbonate alcalin) ou organique (amine tertiaire ou pyridine).

Lorsque l'on opère en milieu organique, l'hydrolyse est réalisée par addition d'eau au mélange réactionnel.

Lorsque l'on opère en présence d'un solvant, il n'est pas nécessaire que le solvant soit miscible à la phase aqueuse acide. Le contact est alors réalisé par agitation vive.

Parmi les solvants utilisables peuvent être cités les solvants chlorés, l'acétate d'éthyle, le tétrahydrofuranne, l'acétonitrile, le diméthylformamide, les alcools.

Il n'est pas absolument nécessaire d'avoir purifié l'intermédiaire de formule générale (XXI) pour mettre en oeuvre cette réaction. Le cas échéant, l'élimination des groupements protecteurs s'effectue dans les conditions décrites précédemment.

Lorsque l'on veut préparer un produit de formule générale (XIX) pour lequel $R°_2$ est un atome d'hydrogène et $R°_1$ est un radical de formule générale (Xa) dans laquelle R" est un groupement de formule générale (II) dont la fonction acide est protégée, il est nécessaire d'utiliser une énamine de formule générale (XXI) dans laquelle les radicaux protecteurs d'acide représentés par $R_2$ et contenu dans R" sont différents et éliminables sélectivement. Il en est de même si l'on veut inversement préparer une cétone de formule générale (XIX) dans laquelle $R°_1$ contient un groupement carboxy libre et $R°_2$ est autre qu'un atome d'hydrogène.

Les produits de formule générale (XIX) pour lesquels n =1 peuvent être obtenus par oxydation du produit de formule générale (XIX) correspondant pour lequel n = 0. On opère par application des méthodes décrites dans la demande de brevet allemand 2 637 176.

Les produits de formule générale (XIX) pour lesquels $R°_1$ est un radical de formule générale (Xa) et $R°_2$, R° et n sont définis comme précédemment peuvent également être obtenus par acylation du produit correspondant de formule générale (XIX) dans laquelle $R°_1$ est un atome d'hydrogène (ou le cas échéant un mélange de ses isomères), au moyen de l'acide de formule générale (X) ou par action d'un dérivé réactif de cet acide, puis éventuellement élimination des radicaux protecteurs.

On opère par analogie avec la méthode décrite précédemment pour la préparation des produits de formule générale (I) à partir des produits de formules générales (X) et (XI).

Il est entendu que, lorsque $R°_1$ contient un radical carboxy, celui-ci est préalablement protégé.

Lorsque $R°_1$ contient un groupement hydroxyimino, celui-ci est libre ou protégé.

A titre d'exemple

les fonctions acides sont protégées par un groupement protecteur tel que défini précédemment pour $R_2$,

l'oxime peut être protégée par un groupement protecteur tel que cité précédemment pour R".

L'élimination de ces radicaux s'effectue dans les conditions décrites précédemment.

Les cétones de formule générale (XIX) dans laquelle $R°_1$ est un atome d'hydrogène peuvent être obtenues par élimination du radical

$R°_1$ d'un produit de formule générale (XIX) dans laquelle $R°_1$ est défini comme $R°_{1a}$ dans la formule générale (XV), ou bien par élimination simultanée des radicaux $R°_1$ et $R°_2$ du produit de formule générale (XIX) tel que défini ci-dessus.

On opère dans les conditions décrites précédemment pour la préparation d'un produit de formule générale (XIV) dans laquelle $R°_{1a}$ est un atome d'hydrogène.

Les produits de formule générale (XXI) pour lesquels $R_1$, $R_2$, $R_7$ et $R_8$ ont les définitions données précédemment, et R° est un radical du premier groupe défini précédemment, peuvent être obtenus par action d'un réactif, éventuellement préparé in situ, de formule générale :

$$R°\text{-}C(NR_7R_8)_3 \qquad (XXII)$$

dans laquelle R°, $R_7$ et $R_8$ sont définis comme ci-dessus, sur un dérivé de céphalosporine de formule générale :

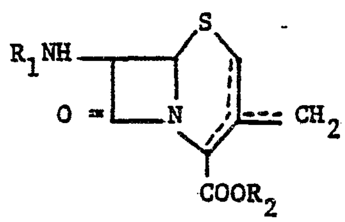

(XXIII)

dans laquelle, $R_1$ et $R_2$ étant définis comme précédemment, le dérivé se présente sous forme méthyl-3 bicyclooctène-2 ou -3 ou méthylène-3 bicyclooctane.

On opère généralement dans un solvant organique tel que le diméthylformamide, l'hexaméthylphosphorotriamide, les glymes, le dioxanne, le diméthylacétamide, l'acétate d'éthyle ou dans un mélange de tels solvants à une température comprise entre 20°C et la température de reflux du mélange réactionnel.

Les produits de formule générale (XXI) dans laquelle $R_1$, $R_2$,

$R_7$ et $R_8$ sont définis comme précédemment et R° est un radical du second groupe défini précédemment peuvent être obtenus par action d'un réactif, éventuellement préparé in situ, de formule générale :

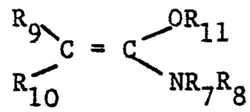

(XXIVa)

ou

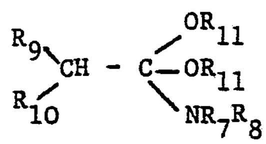

(XXIVb)

(dans lesquelles $R_9$ et $R_{10}$ sont tels que $R_9R_{10}CH-$ représente le radical R° défini ci-dessus, $R_7$ et $R_8$ sont définis comme précédemment et $R_{11}$ est un radical alcoyle contenant 1 à 5 atomes de carbone), sur un dérivé de céphalosporine de formule générale (XXIII).

La réaction s'effectue généralement dans les conditions décrites précédemment pour la préparation des produits de formule générale (XXI) à partir des produits (XXII) et (XXIII).

Les réactifs de formule générale (XXII) peuvent être obtenus selon ou par application de la méthode décrite par C.F. HOBBS et coll., J. Org. Chem. 36, 2885 (1971).

Les réactifs de formule générale (XXIVa) peuvent être obtenus selon ou par application de la méthode décrite par H. BREDERECK et coll., Chem. Ber., 97, 3076 (1964) et Chem. Ber., 97 3081 (1964).

Les réactifs de formule générale (XXIVb) peuvent être obtenus par application de la méthode décrite par H. MERWEIN et coll., Ann., 641, 1 (1961).

Les dérivés de la céphalosporine de formule générale (XXIII) dans laquelle $R_1$ représente un radical de formule générale (Xa) peuvent être préparés à partir des produits de formule générale :

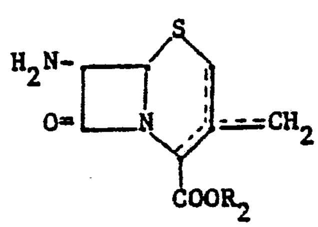

(XXV)

[dans laquelle, $R_2$ étant défini comme précédemment, la position de la double liaison est définie comme pour le produit de formule générale (XXIII)] par action d'un acide de formule générale (X) ou d'un dérivé réactif de cet acide suivie le cas échéant de l'élimination du radical protecteur de l'oxime.

On opère dans les conditions décrites précédemment pour la préparation des produits de formule générale (I) à partir des produits de formule générale (X) et (XI).

L'élimination du radical protecteur de l'oxime s'effectue dans les conditions décrites précédemment.

Les dérivés de la céphalosporine de formule générale (XXIII) dans laquelle $R_1$ est un radical de formule générale (XVII) peuvent être obtenus par application de la méthode décrite par A. MORIMOTO et coll., J.C.S. Perkin I, 1109 (1980) à partir de l'halogénure correspondant $R_1$-Hal [qui peut être lui-même obtenu selon l'une des méthodes décrites par K. SASSE, Methoden den Organischen Chemie Vol. 12, part. 2, p. 274, Houben Weyl, G. Thieme Verlag, Stuttgart (1964)].

L'introduction des groupements protecteurs $R_1$ et/ou $R_2$ des produits de formule générale (XXIII) pour lesquels $R_1$ et $R_2$ sont définis comme précédemment [à l'exception pour $R_1$ de représenter un radical de formule générale (Xa)], et des produits de formule générale (XXIII) pour lesquels $R_2$ est défini comme précédemment, peut être effectuée sur une céphalosporine respectivement de formule générale (XXV) ou de formule générale :

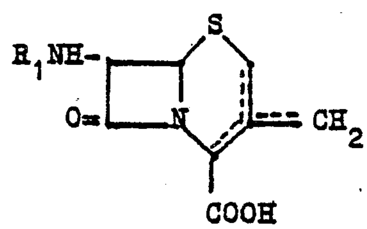

(XXVI)

ou

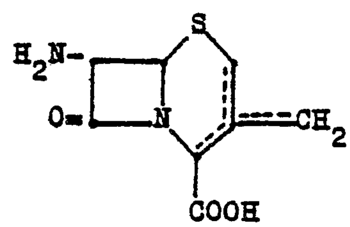

(XXVII)

par application des méthodes décrites dans les références suivantes :

- lorsque $R_1$ est un radical trityle : par analogie avec la méthode décrite par J.C. SHEEHAN et coll., J. Amer. Chem. Soc., 84, 2983 (1962),

- lorsque $R_1$ est phénylacétyle ou phénoxyacétyle : selon E.H. FLYNN, Cephalosporins and Penicillins, Ac. Press (1972),

- lorsque $R_1$ est un radical t.butoxycarbonyle : selon L. MORODER et coll., Hoppe Seyler's Z. Physiol. Chem. 357, 1651 (1976),

- lorsque $R_1$ est benzyloxycarbonyle, p.méthoxybenzyloxycarbonyle, p.nitrobenzyloxycarbonyle ou vinyloxycarbonyle : par action d'un chloroformiate en milieu hydroorganique en présence d'un bicarbonate alcalin, ou selon le brevet belge 788 885.

- lorsque $R_1$ est diphénylméthoxycarbonyle : par action de l'azidoformiate correspondant en milieu hydroorganique, en présence d'un bicarbonate alcalin,

- lorsque $R_1$ est (biphénylyl-4)-2 isopropyloxycarbonyle : par analogie avec la méthode décrite dans Helv. Chim. Acta, 51, 924 (1968),

- lorsque $R_1NH$ est remplacé par diméthylamino-méthylèneamino : par analogie avec la méthode décrite par J.F. FITT, J. Org. Chem. 42(15), 2639 (1977),

- lorsque $R_1NH$ est remplacé par nitro-4 benzylidèneamino ou diméthoxy-3,4 benzylidèneamino : selon la méthode décrite par R.A. FIRESTONE, Tetrahedron Lett., 375 (1972),

- lorsque $R_2$ est méthoxyméthyle : selon S. SEKI et coll., Tetrahedron Lett., 33, 2915 (1977),

- lorsque $R_2$ est t.butyle : selon R.J. STEDMAN, J. Med. Chem., 9, 444 (1966),

- lorsque $R_2$ est benzhydryle : selon la demande de brevet néerlandais 73 03263,

- lorsque $R_2$ est benzyle, nitrobenzyle ou p.méthoxybenzyle : selon R.R. CHAUVETTE et coll., J. Org. Chem., 38(17), 2994 (1973).

Les acides de formule générale (X) dans laquelle R" est hydrogène ou alcoyle peuvent être préparés selon la méthode décrite dans le brevet belge 850 662.

Les produits de formule générale (X) dans laquelle R" est un radical vinyle peuvent être préparés selon la méthode décrite dans le brevet belge 869 079.

Les produits de formule générale (X) dans laquelle R" est un radical cyanométhyle peuvent être préparés selon la méthode décrite dans la demande de brevet allemand 2 812 625.

Les acides de formule générale (X) dans laquelle R" est un radical protecteur peuvent être préparés par protection de l'oxime d'un tel acide dans lequel R" est hydrogène, par toute méthode connue qui n'altère pas le reste de la molécule. La protection s'effectue notamment par les groupements trityle ou tétrahydropyrannyle qui sont éliminables par acidolyse, par exemple par l'acide trifluoracétique, l'acide formique (aqueux ou non) ou l'acide p.toluènesulfonique. La protection peut également s'effectuer par le groupement méthoxy-2 propyle-2 qui peut être éliminé par application de la méthode décrite dans le brevet belge 875 379.

Les acides de formule générale (X) dans laquelle R" est un radical de formule générale (II) peuvent être préparés selon les méthodes décrites dans les brevets belges 864 810, 865 298, 876 541 et 876 542.

Les produits de formule générale (XI) peuvent être obtenus par élimination du radical $R°_{1a}$ d'un produit de formule générale

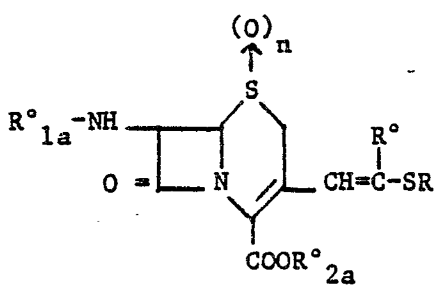

(XXVIII)

dans laquelle R°, R, $R°_{2a}$ et n sont définis comme pour la formule générale (XI) et $R°_{1a}$ est défini comme précédemment pour la formule générale (XV) ou éventuellement élimination simultanée du radical $R°_{1a}$ et des autres radicaux protecteurs de ce produit.

On opère dans les conditions décrites précédemment pour la préparation d'un produit de formule générale (XIV).

Il n'est pas nécessaire d'isoler le produit de formule générale (XI) pour l'utiliser dans la suite de la synthèse.

Les céphalosporines de formule générale (XXVIII) peuvent être préparées par action d'un thiol de formule générale (V) (ou un de ses

sels alcalins ou alcalino-terreux) sur un produit ou un mélange d'isomères du produit de formule générale (XV), puis réduction éventuelle du sulfoxyde obtenu (lorsque n = 1) et élimination éventuelle des radicaux protecteurs de R.

La réaction s'effectue dans les conditions décrites précédemment pour l'obtention d'un produit de formule générale (I) à partir d'un produit de formule générale (VI) et d'un thiol de formule générale (V).

Il est entendu que le radical R du thiol est (le cas échéant) protégé comme décrit précédemment et que l'élimination des radicaux protecteurs peut être effectuée dans les conditions décrites précédemment. Il est cependant préférable de conserver les groupements protecteurs jusqu'à l'obtention du produit de formule générale (I).

Il est souvent préférable d'employer un produit de formule générale (XV) dans laquelle n = 1. Dans le cas contraire lorsque l'on obtient un mélange d'isomères bicyclooctène-2 et bicyclooctène-3, il est nécessaire de transformer le produit en sulfoxyde pour la suite des opérations, ce qui a pour effet de stabiliser la double liaison de telle manière que l'on ait un bicyclooctène-2, puis de réduire le sulfoxyde du produit final obtenu.

Les produits de formules générales (VI), (XV) ou (XXIX), dans lesquelles n = 1, peuvent être obtenus par oxydation des produits correspondants dans lesquels n = 0 selon la méthode décrite dans la demande de brevet allemand 2 637 176.

Les isomères des produits de formules générales (I), (VI), (XI), (XIV), (XV), (XIX), (XXI) ou (XXVIII) peuvent être séparés par chromatographie ou par cristallisation.

Plus particulièrement, les isomères bicyclooctène-2 des produits de formule générale (I), (VI), (XV), (XIX) ou (XXVIII) peuvent être obtenus à partir des mélanges bicyclooctène-2/bicyclooctène-3 par oxydation puis réduction.

Les produits de formule générale (I) peuvent être transformés en sels d'addition avec les acides. Selon les procédés décrits, les produits peuvent être obtenus sous forme de trifluoroacétate, solvate avec l'acide formique ou l'eau, phosphate, méthanesulfonate ou paratoluènesulfonate. Les produits de formule générale (I), obtenus sous forme de ces sels, peuvent être libérés et transformés en sels d'autres acides selon les méthodes habituelles.

Les produits de formule générale (I) peuvent aussi être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration ou décantation. Il peut être obtenu également par lyophilisation de sa solution.

Comme exemples de sels pharmaceutiquement acceptables peuvent être cités les sels d'addition avec des acides minéraux (chlorhydrates, bromhydrates, sulfates, nitrates, phosphates) ou organiques (succinates, fumarates, maléates, p.toluènesulfonates), les sels avec les métaux alcalins (sodium, potassium, lithium) ou avec les métaux alcalino-terreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, dicyclohexylamine, N-benzyl-β-phénéthylamine, NN'-dibenzyléthylènediamine, diphénylènediamine, benzhydrylamine, quinine, choline, arginine, lysine, leucine, dibenzylamine).

Les nouveaux produits selon l'invention et les produits de formules générales (XIX) et (XXI) peuvent être éventuellement purifiés par des méthodes physiques telles que la cristallisation ou la chromatographie.

Les dérivés de la céphalosporine de formule générale (I) et leurs sels pharmaceutiquement acceptables présentent des propriétés antibactériennes particulièrement intéressantes. Ils manifestent une activité remarquable in vitro et in vivo sur les germes Gram-positifs et Gram-négatifs.

In vitro, ils se sont montrés actifs à une concentration comprise entre 2 et 20 µg/cm3 sur des souches de staphylocoques sensibles à la pénicilline G (Staphylococcus aureus Smith), et à une concentration comprise entre 1 et 10 µg/cm3 sur Escherichia coli souche NIHJ.

In vivo, ils se sont montrés actifs sur les infections expérimentales de la souris à Staphylococcus aureus Smith (sensible à la pénicilline G) à une dose comprise entre 1 et 20 mg/kg par jour par voie sous-cutanée, et à Escherichia coli (souche Monod) à des doses comprises entre 0,01 et 10 mg/kg par jour par voie sous-cutanée.

Par ailleurs leur $DL_{50}$ est comprise entre 1,5 et des doses supérieures à 2,5 mg/kg par voie sous-cutanée chez la souris.

D'un intérêt particulier sont les produits de formule générale (I) dans laquelle R est choisi parmi :

pyridyle-2 éventuellement N-oxydé ,

méthyl-6 oxyde-1 pyridazinyle-3,

dioxo-5,6 tétrahydro-1,4,5,6 triazine-1,2,4 yle-3 substitué en position -4 par

un radical alcoyle contenant 1 ou 2 atomes de carbone substitué par un radical alcoyloxy, alcoylthio, formyle,

un radical allyle, dihydroxy-2,3 propyle,

un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, alcoyluréido

thiadiazol-1,3,4 yle-5 substitué par alcoyle, dialcoylaminoalcoyle ou acylaminoalcoyle,

tétrazolyle-5 substitué en position -1 par

un radical alcoyle contenant 1 ou 2 atomes de carbone

un radical alcoyle contenant 2 ou 3 atomes de carbone substitué par hydroxy, acylamino ou dialcoylamino, ou

alcoyl-1 triazol-1,2,4 yle-5 substitué en position -3 par un radical alcoyloxycarbonyle dont les radicaux alcoyle et alcoyloxy contiennent 1 ou 2 atomes de carbone

le symbole R° est un radical alcoyle et le symbole R' est un radical alcoyle.

Plus spécialement intéressants sont les produits de formule générale (I) de forme syn dans laquelle R représente un radical dioxo-5,6 tétrahydro-1,4,5,6 triazine-1,2,4 yle-3 substitué en position -4 par un radical formylméthyle ou formyléthyle, alcoyl-2 thiadiazol-1,3,4 yle-5 ou alcoyl-1 tétrazolyle-5 dont la partie alcoyle contient 1 ou 2 atomes de carbone, et R° et R' représentent des radicaux alcoyle.

Les exemples suivants, donnés à titre non limitatif, illustrent la présente invention.

EXEMPLE 1 -

On refroidit à 4°C une solution de 3,53 g d'amino-7 benzhydryloxycarbonyl-2 {[(méthyl-2 thiadiazol-1,3,4 yl-5) thio]-2 propène-1 yl-1}-3 oxo-8 oxyde-5 thia-5 aza-1 bicyclo[4.2.0] octène-2,7 forme Z dans 250 cm3 de dichlorométhane. On ajoute successivement 3,28 g d'acide méthoxyimino-2 (tritylamino-2 thiazolyl-4)-2 acétique et 1,54 g de dicyclohexylcarbodiimide. On agite le mélange pendant 1 heure 1/2 à 4°C puis concentre à sec sous pression réduite (20 mm de mercure ; 2,7 kPa) à 20°C. Le résidu est repris avec 50 cm3 d'acétate d'éthyle et l'insoluble est séparé par filtration. La solution est lavée avec 100 cm3 de solution aqueuse saturée de bicarbonate de sodium puis avec 100 cm3 d'acide chlorhydrique 0,1N. On sèche sur sulfate de sodium et concentre à sec sous pression réduite (20 mm de mercure ; 2,7 kPa) à 20°C. Le résidu est chromatographié sur une colonne de 80 g de gel de silice Merck (0,06-0,2) (diamètre de la colonne : 3 cm ; hauteur : 64 cm). On élue avec 300 cm3 d'un mélange acétate d'éthyle/cyclohexane 35-65 (en volumes) puis avec 1200 cm3 du mélange acétate d'éthyle/cyclohexane 70-30 (en volumes) en recueillant des fractions de 60 cm3. On concentre à sec les fractions 12 à 20 sous pression réduite (20 mm de mercure ; 2,7 kPa) à 20°C. On obtient ainsi 2 g de benzhydryloxycarbonyl-2 {[(méthyl-2 thiadiazol -1,3,4 yl-5 thio]-2 propène-1 yl-1}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme Z sous la forme d'une meringue blanche.

Spectre infra-rouge (KBr), bandes caractéristiques ($cm^{-1}$) 3420, 1800, 1730, 1685, 1530, 1495, 1450, 1040, 755, 705

Spectre de RMN du proton (350 MHz, $CDCl_3$, δ en ppm, J en Hz) 2,05 (s, 3H, $CH_3$-C=) ; 2,72 (s, 3H, $CH_3$- hétérocycle) ; 3,71 et 4,06 (2d, J = 18, 2H, $-SCH_2-$) ; 4,12 (s, 3H, $=NOCH_3$) ; 4,61 (d, J = 5, 1H, $H_6$) ; 6,21 (dd, J = 5 et 9, 1H, $H_7$) ; 6,73 (s, 1H, -CH=) ; 6,76 (s, 1H, H sur thiazole) ; 6,97 (s, 1H, -COOCH<) ; 7,14 (s large, 1H, -NH trityle) ; 7,44 (d, J = 9, 1H, -CONH-).

Une solution de 2,52 g de benzhydryloxycarbonyl-2 {[(méthyl-2 thiadiazol-1,3,4 yl-5 thio]-2 propène-1 yl-1}-3 [méthoxyimino-2 (tritylamino-2 thiazolyl-4)-2 acétamido]-7 oxo-8 oxyde-5 thia-5 aza-1 bicyclo[4.2.0] octène-2, isomère syn, forme Z dans 40 cm3 de dichlorométhane est refroidie à -10°C. On ajoute successivement 0,95cm3 de N,N-diméthylacétamide et 0,7 g de trichlorure de phosphore. La solution est agitée pendant 20 minutes à -10°C puis versée dans 50 cm3 de solution aqueuse saturée de bicarbonate de sodium. On dilue avec 200 cm3 d'eau distillée et extrait avec 300 cm3 d'acétate d'éthyle. La phase organique est séchée sur sulfate de sodium et concentrée à sec sous pression réduite (20 mm de mercure ; 2,7 kPa) à 20°C. Le résidu est chromatographié sur une colonne de 25 g de gel de silice Merck (0,06-0,20) (diamètre de la colonne : 2 cm ; hauteur : 15 cm). On élue avec 500 cm3 de mélange acétate d'éthyle/cyclohexane 50-50 (en volumes) en recueillant des fractions de 30 cm3. On concentre à sec les fractions 4 à 13 sous pression réduite (20 mm de mercure ; 2,7 kPa) à 20°C. On obtient ainsi 0,77 g de benzhydryloxycarbonyl-2 {[(méthyl-2 thiadiazol -1,3,4 yl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z sous la forme d'une meringue blanche.

Spectre infra-rouge (KBr), bandes caractéristiques ($cm^{-1}$) 1790, 1725, 1685, 1525, 1495, 1450, 1050, 750, 700

Spectre de RMN du proton (350 MHz, $CDCl_3$, δ en ppm, J en Hz) 2,02 (s, 3H, $CH_3$-C=) ; 2,7 (s, 3H, $CH_3$- hétérocycle) ; 3,63 et 3,74 (2d, J = 18, 2H, $-SCH_2-$) ; 4,07 (s, 3H, $=NOCH_3$) ; 5,09 (d, J = 4, 1H, $H_6$) ; 5,94 (dd, J = 4 et 9, 1H, $H_7$) ; 6,6 (s, 1H, -CH= ) ; 6,76 (s, 1H, H thiazole) ; 6,84 (d, J = 9, 1H, -CONH-) ; 6,94 (s, 1H, -COOCH< ) ; 7,04 (s large, 1H, -NH- trityle).

On dissout 0,76 g de benzhydryloxycarbonyl-2 {[(méthyl-2 thiadiazol-1,3,4 yl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z dans 8 cm3 d'acide formique. On chauffe la solution à 50°C et ajoute goutte à goutte 4 cm3 d'eau distillée en 10 minutes. On agite pendant 30 minutes à 50°C puis ajoute 4 cm3 d'eau distillée, refroidit, filtre et concentre la solution à sec sous pression réduite (1 mm de mercure ; 0,13 kPa) à 40°C. On ajoute 40 cm3 d'éthanol qu'on évapore sous pression réduite (1 mm de mercure ; 0,13 kPa) à 30°C et on répète encore 2 fois cette opération. On reprend le résidu avec 10 cm3 d'éthanol, filtre, lave le gâteau avec 20 cm3 d'éther diéthylique , sèche sur sulfate de sodium et évapore le solvant sous pression réduite (1 mm de mercure ; 0,13 kPa) à 20°C. On isole ainsi 60 mg d'[(amino-2 thiazolyl-4)-2 méthoxyimino-2 acétamido]-7 carboxy-2 {[(méthyl-2 thiadiazol -1,3,4 yl-5) thio]-2 propène-1 yl-1}-3 oxo-8 thia-5 aza-1 bicyclo[4.2.0] octène-2, isomère syn, forme Z sous forme d'une poudre jaune.

Spectre infra-rouge (KBr), bandes caractéristiques ($cm^{-1}$) 3600, 2700, 1765, 1670, 1625, 1535, 1365, 1035

Spectre de RMN du proton (350 MHz, DMSO $d_6$, δ en ppm, J en Hz) 2,24 (s large, $=C-CH_3$) ; 2,75 (s, $-CH_3$ thiadiazole) ; 3,87 (s, $=N-OCH_3$) ; 4,51 (d, J = 14, 1H des -H du $-S-CH_2-$) ; 5,44 (dd, J = 5 et 9, -H en 7) ; 5,62 (d, J = 5, -H en 6) ; 6,75 (s, -H thiazole) ; 7,07 (s, -CH=) ; 7,20 (s large, $-NH_2$) ; 9,55 (d, J = 9, -CO-NH-).

L'amino-7 benzhydryloxycarbonyl-2 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z peut être obtenu de la manière suivante :

On chauffe à 40°C une solution de 4,45 g de benzhydryloxycarbonyl-2 t.butoxycarbonylamino-7 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z dans 150 cm3 d'acétonitrile. On ajoute goutte à goutte en 5 minutes une solution de 2,59 g d'acide p.toluènesulfonique (monohydraté) dans 75 cm3 d'acétonitrile. Le mélange est agité à 40°C pendant 35 minutes puis versé dans 200 cm3 de solution aqueuse saturée de bicarbonate de sodium. On ajoute 500 cm3 d'eau distillée et extrait

avec 500 cm3 de dichlorométhane. La phase organique est lavée avec 500 cm3 d'eau distillée, séchée sur sulfate de magnésium et concentrée à sec sous pression réduite (20 mm de mercure ; 2,7 kPa) à 20°C. On obtient ainsi 3,53 g d'amino-7 benzhydryloxycarbonyl-2 {[(méthyl-2 thiadiazol -1,3,4 yl-5) thio]-2 propène-1 yl-1}-3 oxo-8 oxyde-5 thia-5 aza-1 bicyclo[4.2.0] octène-2, forme Z sous la forme d'une meringue brune.

Spectre infra-rouge ($CHCl_3$), bandes caractéristiques ($cm^{-1}$) 3420, 1800, 1725, 1500, 1455, 1370, 1160, 1050, 695.

Le benzhydryloxycarbonyl-2 t.butoxycarbonylamino-7 {[(méthyl-2 thiadiazol-1,3,4 yl-5) thio]-2 propène-1 yl-1}-3 oxo-8 oxyde-5 thia-5 aza-1 bicyclo[4.2.0] octène-2, forme Z peut être préparé de la manière suivante :

On refroidit à 0°C une solution de 0,22 g de benzhydryloxycarbonyl-2 t.butoxycarbonylamino-7 {[(méthyl-2 thiadiazol-1,3,4 yl-5) thio]-2 propène-1 yl-1}-3 oxo-8 thia-5 aza-1 bicyclo[4.2.0] octène-2 et octène-3, forme Z dans 10 cm3 de dichlorométhane. On ajoute goutte à goutte en 20 minutes une solution de 0,07 g d'acide m.chloroperbenzoïque à 85 % dans 5 cm3 de dichlorométhane. On agite ensuite la solution pendant 5 minutes à 0°C, lave avec 20 cm3 de solution saturée de bicarbonate de sodium, sèche sur sulfate de sodium et concentre à sec sous pression réduite (20 mm de mercure ; 2,7 kPa) à 20°C. Le résidu est chromatographié sur une colonne de 4 g de gel de silice Merck (0,06-0,2) (diamètre de la colonne : 1 cm ; hauteur : 10 cm). On élue avec 100 cm3 de mélange acétate d'éthyle/cyclohexane 60-40 (volumes) et recueille des fractions de 3 cm3. On concentre à sec sous pression réduite (20 mm de mercure ; 2,7 kPa) à 20°C les fractions 11 à 22. On obtient ainsi 0,12 g de benzhydryloxycarbonyl-2 t.butoxycarbonylamino-7 {[(méthyl-2 thiadiazol -1,3,4 yl-5) thio]-2 propène-1 yl-1}-3 oxo-8 oxyde-5 thia-5 aza-1 bicyclo[4.2.0] octène-2, forme Z sous forme d'une meringue blanche.

Spectre infra-rouge ($CHCl_3$), bandes caractéristiques ($cm^{-1}$) 3420, 1800, 1720, 1505, 1455, 1370, 1160, 1045, 695

Spectre de RMN du proton (350 MHz, $CDCl_3$, δ en ppm, J en Hz) 1,48 (s, 9H, $-C(CH_3)_3$) ; 2,05 (s, 3H, $-CH_3$) ; 2,72 (s, 3H, $CH_3$-Hétérocycle) ; 3,70 et 4,03 (2d, J = 18, 2H, $-SCH_2-$) ; 4,58 (d, J = 4, 1H, $H_6$) ; 5,77 (d, J = 8, 1H, -CONH-) ; 5,85 (dd, J = 4 et 9, 1H, $H_7$) ; 6,75 (s, 1H, -CH=) ; 6,98 (s, -COOCH<).

Le benzhydryloxycarbonyl-2 t.butoxycarbonylamino-7 {[(méthyl-2 thiadiazol-1,3,4 yl-5) thio]-2 propène-1 yl-1}-3 oxo-8 thia-5 aza-1 bicyclo[4.2.0] octène-2 et octène-3, forme Z peut être préparé de la manière suivante :

On ajoute 1,53 g de sel de sodium de mercapto-5 méthyl-2 thiadiazole-1,3,4 à une solution de 5,9 g de mélange 66-34 (en moles) de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z dans 100 cm3 de diméthylformamide à 20°C. On agite pendant 1 heure 10 minutes à 20°C puis dilue la solution avec 500 cm3 d'eau. On extrait avec 300 cm3 d'acétate d'éthyle, sèche la phase organique sur sulfate de sodium, concentre à sec à 20°C sous pression réduite (20 mm de mercure ; 2,7 kPa) et chromatographie le résidu sur une colonne de 100 g de gel de silice Merck (0,06-0,2) (diamètre de la colonne : 3,5 cm ; hauteur : 25 cm). On élue avec 1 litre de mélange acétate d'éthyle/cyclohexane 35-65 (volumes) et recueille des fractions de 60 cm3. On concentre à sec à 20°C les fractions 8 à 14 sous pression réduite (20 mm de mercure ; 2,7 kPa). On obtient ainsi 1,35 g de mélange 40-60 de benzhydryloxycarbonyl-2 t.butoxycarbonylamino-7 {[(méthyl-2 thiadiazol -1,3,4 yl-5) thio]-2 propène-1 yl-1}-3 oxo-8 thia-5 aza-1 bicyclo[4.2.0] octène-2 et octène-3, forme Z sous forme d'une meringue jaune.

Spectre infra-rouge ($CHCl_3$), bandes caractéristiques ($cm^{-1}$) 3440, 1780, 1720, 1505, 1455, 1390, 1370, 1160

Spectre de RMN du proton (350 MHz, $CDCl_3$, δ en ppm, J en Hz) 1,48 (s, 9H, $-C(CH_3)_3$) ; 2,06 (s large, $CH_3$-C=, octène-2 et -3) ; 2,74 (s, $CH_3$-Het de l'octène-2) ; 2,76 (s, $CH_3$-Het de l'octène-3) ; 3,66 et 3,76 (2d, J = 18, $-SCH_2-$ octène-3) ; 5,04 (d, J = 4, $H_6$ octène-2) ; 5,19 (s, $H_2$ octène-3) ; 5,25 (d, J = 4, $H_6$ octène-3) ; 5,31 (d, J = 8, -CONH- octène-2) ; 5,36 à 5,46 (m, $H_7$ et -CONH- octène-3) ; 5,66 (dd, J = 4 et 8, $H_7$ octène-2) ; 6,18 (s, $H_4$ octène-3) ;

6,58 (s, -CH=C- octène-3) ; 6,64 (s, -CH=C- octène-2) ; 6,90 (s, -COOCH< octène-3) ; 6,97 (s, -COOCH< octène-2).

Le benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z peut être préparé selon la méthode suivante :

A une solution de 6,6 g de tétraméthyl-2,2,6,6 pipéridine dans 50 cm3 de tétrahydrofuranne à 20°C on ajoute en 1 minute 26,5 cm3 de solution 1,6 M de n.butyllithium dans l'hexane. On agite pendant 30 minutes à 20°C puis refroidit le mélange à -70°C. On ajoute goutte à goutte en 20 minutes une solution de 22,2 g d'acétonyl-3 benzhydryloxycarbonyl-2 t.butoxycarbonylamino-7 oxo-8 thia-5 aza-1 bicyclo[4.2.0] octène-2 dans 50 cm3 de tétrahydrofuranne. On agite pendant 30 minutes à -70°C et ajoute 35 cm3 d'hexaméthylphosphorotriamide en 3 minutes puis 10,7 cm3 d'anhydride trifluorométhanesulfonique en 5 minutes. On agite pendant 45 minutes à -70°C puis laisse revenir à 0°C en 1 heure. Le mélange est alors dilué avec 500 cm3 d'acétate d'éthyle et lavé successivement avec 650 cm3 d'acide chlorhydrique 0,2N et 300 cm3 d'eau distillée. La solution est séchée sur sulfate de sodium et concentrée à sec sous pression réduite (20 mm de mercure ; 2,7 kPa) à 20°C. Le résidu est chromatographié sur une colonne de 300 g de gel de silice Merck (0,06-0,2) (diamètre de la colonne : 3,5 cm, hauteur : 70 cm). On élue avec 2 litres de mélange acétate d'éthyle/cyclohexane 20-80 (volumes) et recueille des fractions de 100 cm3. On concentre à sec sous pression réduite (20 mm de mercure ; 2,7 kPa) à 20°C les fractions 3 à 9. On obtient ainsi 6,3 g du mélange 66-34 (moles) des benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2 et octène-3, forme Z sous forme d'une huile jaune.

Spectre infra-rouge ($CHCl_3$), bandes caractéristiques ($cm^{-1}$) 3440, 1790, 1720, 1505, 1455, 1420, 1370, 1155, 1140, 910, 700

Spectre de RMN du proton (350 MHz, $CDCl_3$, δ en ppm, J en Hz) 1,47 (s, 9H, $-C(CH_3)_3$) ; 1,94 (s, 3H, $CH_3-C=$ de l'octène-2) ; 1,99 (s, 3H, $CH_3-C=$ de l'octène-3) ; 3,43 et 3,72 (2d, J = 18, 2H, $-SCH_2-$) ; 5,00 (d, J = 4, $H_6$ octène-2) ; 5,04 (s, $H_2$ de l'octène-3) ;

5,26 (d, J = 4, $H_6$ octène-3) ; 5,27 (d, J = 9, -CONH- octène-2) ; 5,35 à 5,48 (m, 2H, $H_7$ et -CONH- de l'octène-3) ; 5,56 (s, $H_4$ de l'octène-3) ; 5,65 (dd, J = 4 et 9, $H_7$ de l'octène-2) ; 6,17 (s, 1H, -CH=C- de l'octène-2) ; 6,65 (s, 1H, -CH=C- de l'octène-3) ; 6,87 (s, 1H, -COOCH< octène-3) ; 6,95 (s, 1H, -COOCH< de l'octène-2).

L'acétonyl-3 benzhydryloxycarbonyl-2 t.butoxycarbonylamino-7 oxo-8 thia-5 aza-1 bicyclo[4.2.0] octène-2 peut être préparé de la manière suivante :

A une solution de 345 g de benzhydryloxycarbonyl-2 t.butoxycarbonylamino-7 méthyl-3 oxo-8 thia-5 aza-1 bicyclo[4.2.0] octène-2 dans 1600 cm3 de diméthylformamide anhydre à 80°C on ajoute goutte à goutte en 55 minutes 218 g de diméthylamino-1 méthoxy-1 éthylène. On obtient ainsi une solution de benzhydryloxycarbonyl-2 t.butoxycarbonylamino-7 (diméthylamino-2 propène-1 yl-1)-3 oxo-8 thia-5 aza-1 bicyclo[4.2.0] octène-2 que l'on verse dans un mélange agité de 2 litres d'eau distillée, 2 kg de glace et 2,5 litres d'acétate d'éthyle. La phase organique est décantée, lavée avec 3 fois 1 litre d'eau distillée, séchée sur sulfate de sodium et concentrée à sec sous pression réduite (20 mm de mercure ; 2,7 kPa) à 30°C. Le résidu est partagé en 2 parties égales que l'on chromatographie séparément chacune sur une colonne de 1500 g de gel de silice Merck (0,06-0,2) (diamètre de la colonne : 8,5 cm ; hauteur : 70 cm). On élue chaque colonne avec 20 litres de mélange acétate d'éthyle-cyclohexane 23-77 (volumes) en recueillant des fractions de 1 litre. On concentre à sec à 30°C sous pression réduite (20 mm de mercure ; 2,7 kPa) les fractions 7 à 15 de chaque colonne. On obtient ainsi 149 g d'acétonyl-3 benzhydryloxycarbonyl-2 t.butoxycarbonylamino-7 oxo-8 thia-5 aza-1 bicyclo[4.2.0] octène-2 sous la forme d'une meringue jaune.

Spectre infra-rouge ($CHCl_3$), bandes caractéristiques ($cm^{-1}$) 3440, 1780, 1720, 1505, 1455, 1395, 1370, 1160, 695

Spectre de RMN du proton (350 MHz, $CDCl_3$, δ en ppm, J en Hz) 1,48 (s, 9H, $-C(CH_3)_3$) ; 2,10 (s, 3H, $CH_3CO-$) ; 3,29 et 3,62 (2d, J = 19, 2H, $-SCH_2-$) ; 3,57 et 3,67 (2d, J = 16, 2H, $-CH_2CO-$) ; 5,00 (d, J = 4, 1H, $H_6$) ; 5,23 (d, J = 9, 1H, -CONH-) ; 5,62 (dd, J = 4 et 9, 1H, $H_7$) ; 6,97 (s, 1H, -COOCH<).

EXEMPLE 2 -

A une solution de 3,6 g de benzhydryloxycarbonyl-2 t.butoxycarbonylamino-7 {[(méthyl-1 tétrazolyl-5) thio]-2 propène-1 yl-1}-3 oxo-8 thia-5 aza-1 bicyclo[4.2.0] octène-2 forme Z dans 35 cm3 d'acétonitrile chauffée à 40°C on ajoute en 15 minutes une solution de 2,2 g d'acide p.toluènesulfonique dans 20 cm3 d'acétonitrile. Après 1 heure à 40°C, on arrête le chauffage et verse le mélange réactionnel sur un mélange de 300 cm3 de chlorure de méthylène, 500 cm3 d'eau et 50 cm3 d'une solution saturée de bicarbonate de sodium. On agite vivement pendant 5 minutes, sépare la phase organique, la sèche sur sulfate de magnésium, la filtre et la concentre à un volume de 25 cm3 (solution A). Dans le même temps, on met en suspension dans 75 cm3 de toluène 2,57 g d'acide méthoxyimino-2 (tritylamino-2 thiazolyl-4)-2 acétique, concentre à sec sous pression réduite (20 mm de mercure ; 2,7 kPa), ajoute 30 cm3 de chlorure de méthylène anhydre, refroidit la suspension à -5°C, ajoute 0,54 cm3 de N,N diméthylacétamide puis ajoute en 15 minutes, en maintenant le mélange à -5°C, 3,07 cm3 d'une solution 2,1 M de phosgène dans le toluène. Le mélange est maintenu à -5°C pendant 5 heures, puis on ajoute 2,7 g de N,N diméthylacétamide, et, en maintenant la température à -2°C, on ajoute en 15 minutes la solution A. On laisse pendant 1 heure sous agitation à 0°C puis verse le mélange réactionnel sur un mélange agité de 500 cm3 d'acétate d'éthyle, 300 cm3 d'eau et 50 cm3 d'une solution saturée de bicarbonate de sodium. La phase organique est séchée sur sulfate de sodium et concentrée à sec. On obtient 4,4 g d'un produit brut qui est chromatographié sur une colonne de 45 g de gel de silice Merck (0,06-0,2) (diamètre de la colonne : 2,5 cm ; hauteur : 20 cm). On élue avec 800 cm3 d'un mélange acétate d'éthyle/cyclohexane 40-60 (en volumes) en recueillant des fractions de 60 cm3. On concentre à sec les fractions 5 à 12 sous pression réduite (20 mm de mercure ; 2,7 kPa). On obtient ainsi 2,5 g de benzhydryloxycarbonyl-2 {[(méthyl-1 tétrazolyl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z sous forme d'une meringue brun clair.

Spectre infra-rouge (KBr), bandes caractéristiques ($cm^{-1}$) 1785, 1725, 1680, 1530, 1495, 1450, 1220, 755, 700

Spectre de RMN du proton (350 MHz, $CDCl_3$, $\delta$ en ppm, J en Hz) 1,94 (s, 3H, $CH_3$-C=) ; 3,57 et 3,70 (2d, J = 18, 2H, $-SCH_2-$) ; 3,86 (s, 3H, >N-$CH_3$) ; 4,07 (s, 3H, =$NOCH_3$) ; 5,09 (d, J = 4,5, 1H, H en 6) ; 5,97 (dd, J = 4,5 et 8, 1H, H en 7) ; 6,61 (s large, 1H, =CH-) ; 6,75 (s, 1H, H thiazole) ; 6,9 (d, J = 8, 1H, -CONH-) ; 6,94 (s, 1H, -COOCH<).

On dissout 2,46 g de benzhydryloxycarbonyl-2 {[(méthyl-1 tétrazolyl-5) thio]-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z dans 30 cm3 d'acide formique et 14 cm3 d'eau. On chauffe la solution à 50°C, maintient à cette température pendant 30 minutes puis refroidit et filtre le précipité formé. Le filtrat est concentré à sec sous pression réduite (20 mm de mercure ; 2,7 kPa) à 60°C. On ajoute 70 cm3 d'éthanol qu'on évapore sous pression réduite (20 mm de mercure ; 2,7 kPa) et répète cette opération 4 fois. Le résidu est repris par un mélange de 50 cm3 d'acétate d'éthyle et 50 cm3 d'eau contenant 0,46 g de bicarbonate de sodium, puis on agite pendant 3 heures. Les deux phases sont devenues limpides. La phase aqueuse est chromatographiée sur une colonne de 150 cm3 de résine Duolite S 861 (diamètre de la colonne : 2,5 cm) préalablement lavée par 300 cm3 d'une solution à 1 % de chlorure de sodium puis lavée à l'eau jusqu'à disparition des ions chlorures. Après passage de la phase aqueuse sur la colonne, on élue en recueillant des fractions de 60 cm3 par 300 cm3 d'eau puis par 150 cm3 d'une solution aqueuse à 10 % (en volumes) d'éthanol, puis par 200 cm3 d'une solution à 20 % (en volumes) d'éthanol, et enfin par 500 cm3 d'une solution à 30 % (en volumes) d'éthanol. Les fractions 10 à 18 sont réunies, concentrées à demi-volume sous pression réduite (20 mm de mercure ; 2,7 kPa) à 30°C puis lyophilisées. On obtient ainsi 0,64 g du sel de sodium de l'[(amino-2 thiazolyl-4)-2 méthoxyimino-2 acétamido]-7 carboxy-2 {[(méthyl-1 tétrazolyl-5) thio]-2 propène-1 yl-1}-3 oxo-8 thia-5 aza-1 bicyclo[4.2.0] octène-2, isomère syn, forme Z sous forme d'un solide beige clair.

Spectre infra-rouge (KBr), bandes caractéristiques ($cm^{-1}$) 3400 à 3200, 1765, 1665, 1615, 1535, 1390, 1040

Spectre de RMN du proton (350 MHz, DMSO $d_6$, δ en ppm, J en Hz) 1,94 (s, 3H, $CH_3$-C=) ; 3,62 et 3,84 (2d, J = 18, 2H, -S-$CH_2$-) ; 3,84 (s, 3H, $\rangle NCH_3$) ; 4,01 (s, 3H, =$NOCH_3$) ; 5,06 (d, J = 4,5, 1H, H en 6) ; 5,60 (d,d, J = 4,5 et 8, 1H, $H_7$) ; 6,72 (s, 1H, H thiazole) ; 7,10 (s large, 1H, =CH-) ; 7,20 (s large, 2H, -$NH_2$) ; 9,55 (d, J = 8, 1H, -CONH-).

Le benzhydryloxycarbonyl-2 t.butoxycarbonylamino-7 {[(méthyl-1 tétrazolyl-5) thio]-2 propène-1 yl-1}-3 oxo-8 thia-5 aza-1 bicyclo [4.2.0] octène-2, forme Z peut être préparé de la façon suivante :

A une solution de 2,18 g de mercapto-5 méthyl-1 tétrazole dans 140 cm3 de diméthylformamide, on ajoute, à 20°C, 2,94 cm3 de diisopropyléthylamine et 10,4 g de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2, forme Z et on laisse sous agitation pendant 16 heures. Le mélange réactionnel est alors versé sur 300 cm3 d'eau glacée, extrait par deux fois 200 cm3 d'acétate d'éthyle, les phases organiques réunies sont lavées par 200 cm3 d'eau, puis par 100 cm3 d'une solution saturée de chlorure de sodium, séchées sur sulfate de magnésium et concentrées à sec sous pression réduite (20 mm de mercure ; 2,7 kPa) à 30°C. Le résidu (11,1 g) est chromatographié sur une colonne de 175 g de gel de silice Merck (0,06-0,2) (diamètre de la colonne : 3,3 cm ; hauteur : 45 cm). On élue avec 3,5 litres d'un mélange acétate d'éthyle/cyclohexane 80-20 (en volumes) en recueillant des fractions de 100 cm3. Les fractions 23 à 32 sont réunies et concentrées à sec sous pression réduite (20 mm de mercure ; 2,7 kPa) à 30°C. On obtient ainsi 3,6 g de benzhydryloxycarbonyl-2 t.butoxycarbonylamino-7 {[(méthyl-1 tétrazolyl-5) thio]-2 propène-1 yl-1}-3 oxo-8 thia-5 aza-1 bicyclo[4.2.0] octène-2, forme Z sous forme d'une meringue jaune.

Spectre infra-rouge ($CHCl_3$), bandes caractéristiques ($cm^{-1}$) 3440, 1785, 1720, 1505, 1390, 1370, 1160, 700

Spectre de RMN du proton (350 MHz, $CDCl_3$, δ en ppm, J en Hz) 1,48 (s, 9H, $-C(CH_3)_3$) ; 1,94 (s, 3H, $CH_3-C=$) ; 3,55 et 3,70 (2d, J = 18, 2H, $-CH_2S-$) ; 3,87 (s, 3H, $>NCH_3$) ; 5,02 (d, J = 4,5, 1H, $H_6$) ; 5,27 (d, J = 8, 1H, -CONH-) ; 5,67 (dd, J = 4,5 et 8, 1H, $H_7$) ; 6,6 (s, 1H, -CH=C<); 6,95 (s, 1H, -COOCH<).

EXEMPLE 3 -

A une solution de 3,95 g de benzhydryloxycarbonyl-2 t.butoxycarbonylamino-7 {[(diméthoxy-2,2 éthyl)-4 dioxo-5,6 tétrahydro-1,4,5,6 triazine-1,2,4 yl-3] thio-2 propène-1 yl-1}-3 oxo-8 thia-5 aza-1 bicyclo[4.2.0] octène-2, forme Z dans 46,5 cm3 d'acétonitrile et 7 cm3 de méthanol, on ajoute 2,64 cm3 d'acide méthanesulfonique et on porte le mélange pendant 1 heure à 35°C. On verse alors le mélange réactionnel dans un mélange de 500 cm3 de chlorure de méthylène, 200 cm3 d'une solution saturée de bicarbonate de sodium et 300 cm3 d'eau. La phase organique est lavée par 500 cm3 d'eau, séchée sur sulfate de magnésium, filtrée et concentrée jusqu'à un volume de 50 cm3. Cette solution est ajoutée en 25 minutes à 0°C à une solution de chlorure de l'acide méthoxyimino-2 (tritylamino-2 thiazolyl-4)-2 acétique préparée à partir de 2,58 g de l'acide correspondant en opérant comme à l'exemple 2. On laisse ensuite pendant 1 heure à 0°C et verse le mélange réactionnel dans un mélange de 500 cm3 d'acétate d'éthyle, 250 cm3 d'une solution saturée de bicarbonate de sodium et 300 cm3 d'eau. La phase organique est lavée par 30 cm3 d'acide chlorhydrique 4N dilués dans 500 cm3 d'eau, puis par 200 cm3 d'eau, séchée sur sulfate de magnésium, filtrée et concentrée à sec sous pression réduite (20 mm de mercure ; 2,7 kPa) . On obtient ainsi 4,45 g d'un résidu que l'on chromatographie sur une colonne de 60 g de gel de silice Merck (0,06-0,2) (diamètre de la colonne : 2,5 cm ; hauteur : 25 cm). On élue par 700 cm3 d'un mélange acétate d'éthyle/cyclohexane 40-60 (en volumes) en recueillant des fractions de 30 cm3. Les fractions 13 à 20 sont réunies et concentrées à sec sous pression réduite. On obtient ainsi 1,45 g de benzhydryloxycarbonyl-2 {[(diméthoxy-2,2 éthyl)-4 dioxo-5,6

tétrahydro-1,4,5,6 triazine-1,2,4 yl-3] thio-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z sous forme d'une meringue jaune.

Spectre infra-rouge (KBr), bandes caractéristiques ($cm^{-1}$) 3400, 3300, 1790, 1725, 1695, 1585, 1530, 1500, 1450, 1080, 1045, 755, 700

Spectre de RMN du proton (350 MHz, DMSO $d_6$, δ en ppm, J en Hz) 1,90 (s, 3H, $CH_3$-C=) ; 3,32 (s, 6H, $-CH(OCH_3)_2$) ; 3,65 et 3,74 (2d, J = 18, 2H, $-SCH_2-$) ; 3,81 (s, 3H, $=NOCH_3$) ; 3,95 (d, J = 5, 2H, >N-$CH_2$-) ; 4,53 (t, J = 5, 1H, CH<(O-)(O-)) ; 5,21 (d, J = 4,5, 1H, $H_6$) ; 5,76 (dd, J = 4,5 et 9, 1H, $H_7$) ; 6,59 (s, 1H, -CH=) ; 6,72 (s, 1H, H thiazole) ; 6,88 (s, 1H, -COOCH<) ; 8,82 (s, 1H, NHC≡) ; 9,60 (d, J = 9, 1H, -CONH-) ; 12,61 (s, 1H,-NH- triazine).

On dissout 1,40 g de benzhydryloxycarbonyl-2 {[(diméthoxy-2,2 éthyl)-4 dioxo-5,6 tétrahydro-1,4,5,6 triazine-1,2,4 yl-3] thio-2 propène-1 yl-1}-3 [méthoxyimino-2 (tritylamino-2 thiazolyl-4)-2 acétamido]-7 oxo-8 thia-5 aza-1 bicyclo[4.2.0] octène-2, isomère syn, forme Z dans 42 cm3 d'acide formique, porte la solution à 50°C pendant 30 minutes, ajoute alors 4,2 cm3 d'eau et laisse encore pendant 10 minutes à 50°C. Le mélange est refroidi, filtré et concentré à sec à 50°C sous pression réduite (20 mm de mercure ; 2,7 kPa). Le résidu est repris par 50 cm3 d'acétone qu'on évapore à sec sous pression réduite (20 mm de mercure ; 2,7 kPa) et répète cette opération 3 fois. Pour finir, on ajoute 50 cm3 d'acétone, agite pendant 10 minutes et filtre. Après avoir séché, on obtient 0,7 g d'une poudre jaune que l'on dissout dans 20 cm3 d'eau contenant 0,193 g de bicarbonate de sodium. Cette solution est passée sur une colonne de 100 cm3 de résine Duolite S 861 (diamètre de la colonne : 2 cm) qui a été préalablement lavée par 200 cm3 d'une solution à 1 % de chlorure de sodium puis par de l'eau distillée jusqu'à disparition des ions chlorures. On élue par 270 cm3 d'eau, puis par 100 cm3 d'une solution aqueuse à 10 % (en volumes) d'éthanol, puis par 100 cm3 d'une solution à 20 % (en volumes) d'éthanol et enfin par 800 cm3 d'une solution à 30 % (en volumes)

d'éthanol en recueillant des fractions de 75 cm3. Les fractions 10 à 16 sont réunies, concentrées à demi-volume sous pression réduite (20 mm de mercure ; 2,7 kPa) et lyophilisées. On obtient ainsi 0,37 g du sel de sodium de l'[(amino-2 thiazolyl-4)-2 méthoxyimino-2 acétamido]-7 carboxy-2 {[dioxo-5,6 (oxo-2 éthyl)-4 tétrahydro-1,4,5,6 triazine-1,2,4 yl-3] thio-2 propène-1 yl-1}-3 oxo-8 thia-5 aza-1 bicyclo[4.2.0] octène-2, isomère syn, forme Z sous forme d'un solide beige clair.

Spectre infra-rouge (KBr), bandes caractéristiques ($cm^{-1}$) 3420, 1765, 1710, 1680, 1600, 1535, 1390, 1040

Spectre de RMN du proton (350 MHz, $D_2O$, δ en ppm, J en Hz) 2,16 (s, 3H, $CH_3$-C=) ; 3,55 et 3,72 (2d, J = 18, 2H, $-SCH_2-$) ; 4,02 (s, 3H, $=NOCH_3$) ; 5,30 (d, J = 4, 1H, $H_6$) ; 5,38 (s large, 2H, $>NCH_2-$) ; 5,85 (d, J = 4, 1H, $H_7$) ; 6,61 (s, 1H, H thiazole) ; 7,06 (s, 1H, -CH=C<).

Le benzhydryloxycarbonyl-2 t.butoxycarbonylamino-7 {[(diméthoxy-2,2 éthyl)-4 dioxo-5,6 tétrahydro-1,4,5,6 triazine-1,2,4 yl-3] thio-2 propène-1 yl-1}-3 oxo-8 thia-5 aza-1 bicyclo[4.2.0] octène-2, forme Z peut être préparé de la façon suivante :

A une solution de 4,36 g de (diméthoxy-2,2 éthyl)-4 dioxo-5,6 mercapto-3 tétrahydro-1,4,5,6 triazine-1,2,4 et de 2,85 cm3 de diisopropyléthylamine dans 120 cm3 de diméthylformamide on ajoute à environ 20°C 10 g de benzhydryloxycarbonyl-2 t.butoxycarbonylamino-7 oxo-8 (trifluorométhanesulfonyloxy-2 propène-1 yl-1)-3 thia-5 aza-1 bicyclo[4.2.0] octène-2, forme Z et on laisse sous agitation pendant 17 heures. Le mélange réactionnel est alors versé sur 500 cm3 d'eau glacée, extrait par deux fois 200 cm3 d'acétate d'éthyle, les phases organiques réunies sont lavées par trois fois 300 cm3 d'eau, séchées sur sulfate de sodium, filtrées et concentrées à sec sous pression réduite (20 mm de mercure ; 2,7 kPa). Le résidu (10,2 g) est chromatographié sur une colonne de 100 g de gel de silice Merck (0,06-0,2) (diamètre de la colonne : 3 cm ; hauteur : 30 cm). On élue d'abord par 300 cm3 d'un mélange acétate d'éthyle/cyclohexane 30-70 (en volumes)

puis par 600 cm3 d'un mélange acétate d'éthyle/cyclohexane 70-30 (en volumes) en recueillant des fractions de 60 cm3. Les fractions 8 à 14 sont réunies et concentrées à sec sous pression réduite (20 mm de mercure ; 2,7 kPa). On obtient ainsi 4 g de benzhydryloxycarbonyl-2 t.butoxycarbonylamino-7 {[(diméthoxy-2,2 éthyl)-4 dioxo-5,6 tétrahydro-1,4,5,6 triazine-1,2,4 yl-3] thio-2 propène-1 yl-1}-3 oxo-8 thia-5 aza-1 bicyclo[4.2.0] octène-2, forme Z sous forme d'une meringue jaune.

Spectre de RMN du proton (350 MHz, $CDCl_3$, δ en ppm, J en Hz) 1,48 (s, 9H, $-C(CH_3)_3$) ; 2,0 (s, 3H, $CH_3-C=$) ; 3,4 (s, 6H, $-CH(OCH_3)_2$) ; 3,57 et 3,63 (2d, J = 18, 2H, $-SCH_2-$) ; 4 à 4,15 (m, 2H, $>NCH_2-$) ; 4,64 (t, J = 5, 1H, $-CH{<}^{O-}_{O-}$ ) ; 5,01 (d, J = 5, 1H, $H_6$) ; 5,32 (d, J = 9,5, 1H, -CONH-) ; 5,64 (dd, J = 5 et 9,5, 1H, $H_7$) ; 6,67 (s, 1H, -CH=) ; 6,92 (s, 1H, -COOCH< ) ; 10,81 (s, 1H, -NH-triazine).

En opérant de manière analogue, on peut utiliser le produit des exemples de référence ci-dessous pour préparer un produit selon l'invention.

<u>EXEMPLE DE REFERENCE 1</u>

A une solution de 7,6 g de benzhydryloxycarbonyl-2 t.butoxycarbonylamino-7 méthyl-3 oxo-8 thia-5 aza-1 bicyclo[4.2.0] octène-2 dans 50 cm3 de dioxanne à 80°C, on ajoute en 8 minutes une solution de 7 g de tris-diméthylaminométhylbenzène dans 50 cm3 de dioxanne et on maintient à 80°C pendant 12 minutes sous agitation. A ce stade on obtient un mélange de benzhydryloxycarbonyl-2 t.butoxycarbonylamino-7 [α-(β-diméthylaminostyryl)]-3 oxo-8 thia-5 aza-1 bicyclo[4.2.0] octène-2 et octène-3, formes Z et E. Le mélange réactionnel est versé dans un mélange agité de 200 cm3 d'acétate d'éthyle, de 20 cm3 d'acide chlorhydrique 1N et de 20 g de glace. L'agitation est continuée à 20°C pendant 22 heures. La phase organique est séparée, lavée par 50 cm3 d'une solution saturée de bicarbonate de sodium, puis 2 fois par 100 cm3 d'eau distillée, séchée sur sulfate de magnésium et évaporée à sec sous pression réduite (20 mm de mercure ; 2,7 kPa) à 20°C. Le résidu (11,2 g) est dissous dans 100 cm3 d'un mélange acétate d'éthyle/cyclohexane 30-70 (en volumes). La solution est alors filtrée sur 50 g de gel de silice

Merck (0,06-0,2), lavée par 200 cm3 du même mélange et le filtrat évaporé. On obtient 8,3 g d'un mélange de benzhydryloxycarbonyl-2 t.butoxycarbonylamino-7 oxo-8 phénacyl-3 thia-5 aza-1 bicyclo[4.2.0] octène-2 et octène-3 sous forme d'une meringue blonde.

Spectre infra-rouge ($CHBr_3$), bandes caractéristiques ($cm^{-1}$) 3420, 1785, 1720, 1690, 1595, 1580, 1505, 1495, 1455, 1450, 1390, 1370, 760, 745

Spectre de RMN du proton (350 MHz, $CDCl_3$, δ en ppm, J en Hz) Mélange 50-50 d'octène-2 et d'octène-3

1,45 (s, 9H, $-C(CH_3)_3$ octène-2 + octène-3) ; 3,32 et 3,67 (2d, J = 18, 1H, $-SCH_2-$ octène-2) ; 3,69 et 3,92 (2d, J = 17, 1H, $-CH_2CO-$ octène-3 ) ; 4,08 et 4,46 (2d, J = 17, 1H, $-CH_2CO-$ octène-2) ; 5,07 (d, J = 5, 0,5 H, H en 6 octène-2) ; 5,24 (d, J = 4, 0,5 H, H en 6 octène-3) ; 5,30 (s, 0,5 H, H en 2 octène-3) ; 5,20 à 5,35 (m, 0,5 H, -CONH- octène-2) ; 5,35 à 5,50 (m, 1H, H en 7 et -CONH- octène-3) ; 5,64 (d,d, J = 5 et 9, 0,5 H, -H en 7 octène-2) ; 6,14 (s, 0,5 H, H en 4 octène-3) ; 6,86 (s, 1H, -COOCH< octène-2 et octène-3).

A une solution de 4,5 g de benzhydryloxycarbonyl-2 t.butoxycarbonylamino-7 oxo-8 phénacyl-3 thia-5 aza-1 bicyclo[4.2.0] octène-2 et octène-3 dans 90 cm3 de dichlorométhane à 5°C, on ajoute 1,6 g d'acide m.chloroperbenzoïque à 85 % et laisse 30 minutes à 5°C sous agitation. Le mélange réactionnel est versé sur 50 cm3 d'une solution saturée de bicarbonate de sodium ; la phase organique est lavée par 50 cm3 d'eau distillée, séchée sur sulfate de magnésium et évaporée à sec sous pression réduite (20 mm de mercure ; 2,7 kPa) à 20°C. Le résidu (4,25 g) est fixé sur 20 g de gel de silice Merck (0,06-0,2) et chromatographié sur une colonne de 150 g de gel de silice Merck (0,06-0,2) (diamètre de la colonne : 3,6 cm ; hauteur : 40 cm). On élue d'abord par 700 cm3 d'un mélange acétate d'éthyle/cyclohexane 80-20 (en volumes) puis par 1 litre d'un mélange acétate d'éthyle/cyclohexane 70-30 (en volumes),par 500 cm3 d'un mélange 65-35 , par 500 cm3 d'un mélange 60-40 et enfin par 1 litre d'un mélange 50-50 en recueillant des fractions de 50 cm3. Les fractions 47 à 56 sont réunies et le solvant est évaporé à sec sous pression réduite (20 mm de mercure ; 2,7 kPa) à 20°C. On obtient ainsi 2,5 g de benzhydryloxycarbonyl-2 t.butoxycarbonylamino-7 oxo-8 oxyde-5 phénacyl-3 thia-5 aza-1 bicyclo[4.2.0] octène-2 sous forme d'une meringue brune.

Spectre infra-rouge ($CHCl_3$), bandes caractéristiques ($cm^{-1}$) 3410, 1800, 1715, 1685, 1595, 1580, 1500, 1445, 1390, 1370, 1165, 1040

Spectre de RMN du proton (350 MHz, $CDCl_3$, δ en ppm, J en Hz) 1,50 (s, 9H, $(CH_3)_3C-$) ; 3,62 (s, 2H, $-CH_2CO-$) ; 3,65 et 5,28 (2d, J = 18, 2H, $-CH_2S\rightarrow O$) ; 4,65 (d, J = 4, 1H, H en 6) ; 5,82 (s large, 2H, H en 7 et -CONH-) ; 6,86 (s, 1H, -COOCH<).

EXEMPLE DE REFERENCE 2

A une solution de 8,15 g d'acide p.toluènesulfonique monohydraté dans 170 cm3 d'acétonitrile tiédie à 35°C on ajoute 10,1 g de benzhydryloxycarbonyl-2 t.butoxycarbonylamino-7 oxo-8 oxyde-5 phénacyl-3 thia-5 aza-1 bicyclo[4.2.0] octène-2 et laisse à 35°C sous agitation pendant 1 heure. Le mélange réactionnel est alors versé sur un mélange agité de 150 cm3 d'une solution saturée de bicarbonate de sodium et de 300 cm3 d'acétate d'éthyle. La phase organique est ensuite lavée par 100 cm3 d'eau distillée, séchée sur sulfate de magnésium et évaporée à sec sous pression réduite (20 mm de mercure ; 2,7 kPa) à 20°C. On obtient ainsi 7 g d'amino-7 benzhydryloxycarbonyl-2 oxo-8 oxyde-5 phénacyl-3 thia-5 aza-1 bicyclo[4.2.0] octène-2 brut sous forme d'une huile épaisse utilisable sans purification.

Une solution de 1,25 g d'amino-7 benzhydryloxycarbonyl-2 oxo-8 oxyde-5 phénacyl-3 thia-5 aza-1 bicyclo[4.2.0] octène-2 et de 1,05 cm3 de triéthylamine dans 10 cm3 de dichlorométhane refroidie à 0°C est ajoutée en 1 minute à une solution de chlorure de méthoxy-2 (tritylamino-2 thiazolyl-4)-2 acétyle (préparé à partir de 1,21 g d'acide méthoxyimino-2 (tritylamino-2 thiazolyl-4)-2 acétique, 1,3 cm3 d'une solution 2,4 M de phosgène dans du chlorobenzène, 0,23 cm3 de diméthylacétamide dans 15 cm3 de dichlorométhane pendant 16 heures à -7°C) à -10°C. Le mélange réactionnel est agité, pendant 1 heure à -5°C, puis 1 heure en laissant remonter la température de -5°C à 20°C, puis versé sur 5 cm3 d'acide chlorhydrique 0,5 N. La phase organique est lavée par 10 cm3 d'eau, séchée sur sulfate de magnésium et évaporée à sec sous pression réduite (20 mm de mercure ; 2,7 kPa) à 20°C. Le résidu est solidifié dans 20 cm3 d'éther isopropylique. On obtient de cette façon 2 g de benzhydryloxycarbonyl-2 [méthoxyimino-2 (tritylamino-2 thiazolyl-4)-2 acétamido]-7 oxo-8

oxyde-5 phénacyl-3 thia-5 aza-1 bicyclo[4.2.0] octène-2, isomère syn sous forme d'un solide orangé.

Spectre infra-rouge ($CHCl_3$), bandes caractéristiques ($cm^{-1}$) 3400, 1800, 1725, 1680, 1590, 1580, 1510, 1490, 1445, 1040, 690

Spectre de RMN du proton (350 MHz, $CDCl_3$, δ en ppm, J en Hz) 3,59 (s large, 2H) ; 3,68 et 5,22 (2d, J = 18, 2H, $-CH_2S-$ et $-CH_2CO-$) ; 4,09 (s, 3H, $=NOCH_3$) ; 4,71 (d, J = 5, 1H, H en 6) ; 6,16 (dd, J = 5 et 9, 1H, H en 7) ; 6,74 (s, 1H, H thiazole) ; 6,85 (s, 1H, -COOCH<) ; 7,07 (s large, 1H, $-N\underline{H}C(C_6H_5)_3$) ; 7,50 (d, J = 9, 1H, -CONH-).

A une solution de 4,3 g de benzhydryloxycarbonyl-2 [méthoxyimino-2 (tritylamino-2 thiazolyl-4)-2 acétamido]-7 oxo-8 oxyde-5 phénacyl-3 thia-5 aza-1 bicyclo[4.2.0] octène-2, isomère syn et de 1,88 cm3 de diméthylacétamide dans 43 cm3 de dichlorométhane refroidie à -8°C, on ajoute en 1 minute 0,81 cm3 de trichlorure de phosphore et laisse pendant 1 heure sous agitation à -8°C. Le mélange est alors versé sur 20 cm3 d'une solution saturée de bicarbonate de sodium et 100 cm3 d'acétate d'éthyle. La phase organique est lavée par 30 cm3 d'eau, séchée sur sulfate de magnésium et évaporée à sec sous pression réduite (20 mm de mercure ; 2,7 kPa) à 20°C.Le résidu est dissous dans 15 cm3 d'acétate d'éthyle, la solution est filtrée sur 13 g de gel de silice Merck (0,06-0,2) et lavée par 20 cm3 d'acétate d'éthyle. Le filtrat est évaporé à sec sous pression réduite (20 mm de mercure ; 2,7 kPa) à 20°C. On obtient ainsi 3,6 g de benzhydryloxycarbonyl-2 [méthoxyimino-2 (tritylamino-2 thiazolyl-4)-2 acétamido]-7 oxo-8 phénacyl-3 thia-5 aza-1 bicyclo[4.2.0] octène-2, isomère syn sous forme d'un solide beige.

Spectre infra-rouge ($CHCl_3$), bandes caractéristiques ($cm^{-1}$) 3400, 2820, 1780, 1725, 1680, 1590, 1580, 1520, 1490, 1450, 1040

Spectre de RMN du proton (350 MHz, $CDCl_3$, δ en ppm, J en Hz) 3,30 et 3,63 (2d, J = 18, 2H, $-CH_2S-$) ; 4,07 (s, 3H, $=NOCH_3$) ; 4,11 et 4,40 (2d, J = 18, 2H, $-CH_2CO-$) ; 5,11 (d, J = 5, 1H, H en 6 ) ; 5,95 (dd, J = 5 et 9, 1H, H en 7) ; 6,77 (s, 1H, H du thiazole) ; 6,87 (s, 1H, -COOCH<) ; 6,91 (d, J = 9, 1H, -CONH-) ; 7,03 (s, 1H, $-N\underline{H}C(C_6H_5)_3$).

On dissout 2,7 g de benzhydryloxycarbonyl-2 [méthoxyimino-2 (tritylamino-2 thiazolyl-4)-2 acétamido]-7 oxo-8 phénacyl-3 thia-5 aza-1 bicyclo[4.2.0] octène-2, isomère syn dans 55 cm3 d'acide formique à 20°C. On ajoute 11 cm3 d'eau distillée et chauffe le mélange pendant 15 minutes à 45°C. Le mélange est refroidi à 20°C, dilué par 44 cm3 d'eau et filtré. Le filtrat est amené à sec sous pression réduite (5 mm de mercure ; 0,67 kPa) à 20°C. Le résidu est repris 3 fois par 50 cm3 d'éthanol en évaporant à chaque fois le solvant sous pression réduite (5 mm de mercure ; 0,67 kPa) à 20°C pour éliminer l'eau et l'acide formique. Le résidu final est repris par 50 cm3 d'une solution saturée de bicarbonate de sodium, la phase aqueuse est lavée par 50 cm3 d'acétate d'éthyle et acidifiée à pH 3 par de l'acide chlorhydrique 1N sous agitation en présence de 100 cm3 d'acétate d'éthyle. La phase organique est séchée sur sulfate de magnésium et évaporée à sec sous pression réduite (20 mm de mercure ; 2,7 kPa) à 20°C. On obtient ainsi 0,54 g d'[(amino-2 thiazolyl-4)-2 méthoxyimino-2 acétamido]-7 carboxy-2 oxo-8 phénacyl-3 thia-5 aza-1 bicyclo[4.2.0] octène-2, isomère syn sous forme d'un solide beige.

Spectre infra-rouge (KBr), bandes caractéristiques ($cm^{-1}$) 3340, 3250 à 2200, 1770, 1675, 1630, 1595, 1580, 1530, 1445, 1040, 690

Spectre de RMN du proton (350 MHz, DMSO $d_6$, δ en ppm, J en Hz) 3,45 et 3,63 (2d, J = 18, 2H, $-CH_2S-$) ; 3,85 (s, 3H, $=N-OCH_3$) ; 4,20 et 4,45 (2d, J = 18, 2H, $-CH_2CO-$) ; 5,17 (d, J = 5, 1H, H en 6) ; 5,75 (dd, J = 5 et 9, 1H, H en 7) ; 6,75 (s, 1H, H thiazole) ; 7,2 (s, 2H, $-NH_2$) ; 9,65 (d, J = 9, 1H, -CONH-).

Les produits de formule générale (I) sont utilisables dans des médicaments qui les contiennent comme produit actif à l'état pur (sous forme libre ou sous forme de sel) ou sous forme d'une composition en association avec un ou plusieurs adjuvants pharmaceutiquement acceptables. Ces médicaments peuvent être utilisés par voie orale, parentérale ou rectale.

Comme compositions solides pour administration orale peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui seront dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou un glycéride semi-synthétique.

En thérapeutique humaine, ces médicaments sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 1 et 10 g par jour de produit actif par voie orale, intramusculaire ou intraveineuse pour un adulte.

L'exemple suivant, donné à titre non limitatif, illustre une composition pharmaceutique contenant un produit de formule générale (I).

EXEMPLE -

On prépare une solution injectable ayant la composition suivante :

- sel de sodium de l'[(amino-2 thiazolyl-4)-2 méthoxyimino-2 acétamido]-7 carboxy-2 {[(méthyl-1 tétrazolyl-5) thio]-2 propène-1 yl-1}-3 oxo-8 thia-5 aza-1 bicyclo[4.2.0] octène-2, isomère syn, forme Z ................................ 260 mg
- chlorure de sodium ................................ 1,5 mg
- soluté injectable ................................ 2 cm3

Cette solution contient 250 mg de produit actif compté en acide libre.

## REVENDICATIONS

1 - Un nouveau dérivé de la céphalosporine de formule générale :

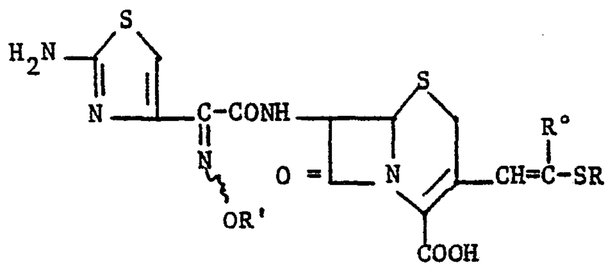

dans laquelle

R est choisi parmi les significations

1) pyridyle-2, -3 ou -4 éventuellement N-oxydés

2) pyrimidinyle-2

3) méthyl-6 oxyde-1 pyridazinyle-3

4) dioxo-5,6 tétrahydro-1,4,5,6 triazine-1,2,4 yle-3 substitué en position -4 par

a) un radical alcoyle contenant 1 ou 2 atomes de carbone éventuellement substitué par un radical alcoyloxy, alcoylthio, formyle,

b) un radical allyle, dihydroxy-2,3 propyle, dihydroxy-1,3 propyle-2 ou formyl-2 hydroxy-2 éthyle,

c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,

5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle

6) alcoyl-2 dihydro-2,5 oxo-5 triazine-1,2,4 yle-3 éventuellement

substitué en position -6 par un radical alcoyle ou alcoyloxy dont les portions et radicaux alcoyles contiennent 1 ou 2 atomes de carbone,

7) amino-1 dihydro-1,2 oxo-2 pyrimidinyle-4

8) thiadiazol-1,3,4 yle-5 substitué par alcoyle, dialcoylaminoalcoyle ou acylaminoalcoyle,

9) tétrazolyle-5 substitué en position -1 par

a) un radical alcoyle contenant 1 ou 2 atomes de carbone éventuellement substitué par un radical formyle,

b) un radical alcoyle contenant 2 ou 3 atomes de carbone substitué par hydroxy, acylamino, ou dialcoylamino, ou

10) a) alcoyl-1 triazol-1,2,4 yle-5 éventuellement substitué en position -3 par un radical alcoyloxycarbonyle dont les radicaux alcoyle et alcoyloxy contiennent 1 ou 2 atomes de carbone

b) alcoyl-1 triazol-1,3,4 yle-5,

- R° représente un radical d'un premier groupe constitué par un radical phényle éventuellement substitué [par un radical alcoyle, trifluorométhyle, dialcoylaminométhyle, alcoyloxy, alcoylthio, dialcoylamino ou par un atome d'halogène tel que le fluor ou le chlore], un radical hétérocyclyle à 5 ou 6 chaînons contenant 1 seul hétéroatome tel que pyridyle-2 ou -3, thiényle-2 ou -3 ou furyle-2 ou -3 éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons, ou un radical hétérocyclyle à 5 ou 6 chaînons contenant un atome d'oxygène ou de soufre et

R' représente un atome d'hydrogène, un radical alcoyle, vinyle, cyanométhyle ou un radical de formule générale

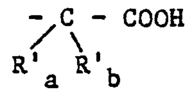

(dans laquelle $R'_a$ et $R'_b$ qui sont identiques ou différents sont des atomes d'hydrogène, des radicaux alcoyle, ou forment ensemble un radical alcoylène contenant 2 ou 3 atomes de carbone), étant entendu que les substituants R° et -SR peuvent être situés en position E ou Z par rapport au noyau céphalosporine, que le radical -OR' peut se trouver en position syn ou anti et que les radicaux alcoyle et acyle cités ci-dessus contiennent (sauf mention spéciale) 1 à 4 atomes de carbone et sont droits ou ramifiés, ainsi que ses sels d'addition avec les acides, ses sels métalliques et ses sels d'addition avec les bases azotées.

2 - Un nouveau dérivé selon la revendication 1, caractérisé en ce que le symbole R représente un radical dioxo-5,6 tétrahydro-1,4,5,6 triazine-1,2,4 yle-3 substitué en position -4 par un radical formylméthyle ou formyléthyle, alcoyl-2 thiadiazol-1,3,4 yle-5 ou alcoyl-1 tétrazolyle-5 dont la partie alcoyle contient 1 ou 2 atomes de carbone, le symbole R° représente un radical alcoyle et le symbole R' représente un radical alcoyle, sous sa forme syn, ainsi que ses sels d'addition avec les acides, ses sels métalliques et ses sels d'addition avec les bases azotées.

3 - L'[(amino-2 thiazolyl-4)-2 méthoxyimino-2 acétamido]-7 carboxy-2 {[(méthyl-2 thiadiazol-1,3,4 yl-5) thio]-2 propène-1 yl-1}-3 oxo-8 thia-5 aza-1 bicyclo[4.2.0] octène-2, isomère syn, forme Z.

4 - Le sel de sodium de l'[(amino-2 thiazolyl-4)-2 méthoxyimino-2 acétamido]-7 carboxy-2 {[(méthyl-1 tétrazolyl-5) thio]-2 propène-1 yl-1}-3 oxo-8 thia-5 aza-1 bicyclo[4.2.0] octène-2, isomère syn, forme Z.

5 - Le sel de sodium de l'[(amino-2 thiazolyl-4)-2 méthoxyimino-2 acétamido]-7 carboxy-2 {[dioxo-5,6 (oxo-2 éthyl)-4 tétrahydro-1,4,5,6 triazine-1,2,4 yl-3] thio-2 propène-1 yl-1}-3 oxo-8 thia-5 aza-1 bicyclo[4.2.0] octène-2, isomère syn, forme Z.

6 - Un procédé de préparation d'un produit selon la revendication 1 caractérisé en ce que l'on fait agir un thiol de formule générale

R - SH

(ou un de ses sels alcalins ou alcalinoterreux), [dans laquelle le substituant R, qui est défini comme dans la revendication 1, est protégé à l'état d'acétal lorsque l'on veut obtenir un produit selon la revendication 1 pour lequel R contient un radical formyle], sur un dérivé de la céphalosporine (ou un mélange des isomères) de formule générale

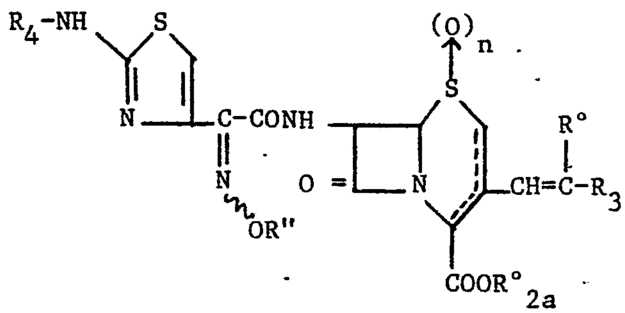

[dans laquelle R° est défini comme dans la revendication 1, R" représente un atome d'hydrogène, un radical alcoyle, vinyle, cyanométhyle, un groupement protecteur d'oxime ou un radical de formule générale :

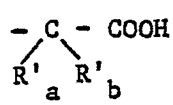

(dans laquelle $R'_a$ et $R'_b$ sont définis comme dans la revendication 1 et dont la fonction acide peut être protégée), et $R_4$ représente un atome d'hydrogène ou un radical protecteur d'amine], $R°_{2a}$ représente un atome d'hydrogène ou un radical protecteur d'acide et le symbole $R_3$ représente un radical de formule générale :

$R'_3\ SO_2O-$

dans laquelle $R'_3$ représente un radical alcoyle, trihalogénométhyle, ou phényle éventuellement substitué par un atome d'halogène ou par un ou plusieurs radicaux alcoyle ou nitro, et n égale 0 ou 1, (étant entendu que lorsque n = 0, le produit se présente sous forme bicyclooctène-2 ou -3 et lorsque n = 1, le produit se présente sous forme bicyclooctène-2), puis lorsque n = 1, on réduit le produit obtenu, élimine s'il y a lieu les radicaux protecteurs et transforme éventuellement le produit obtenu en sel d'addition avec un acide, en sel métallique ou en sel d'addition avec une base azotée.

7 - Un procédé de préparation d'un produit selon la revendication 1, caractérisé en ce que l'on fait agir un acide de formule générale :

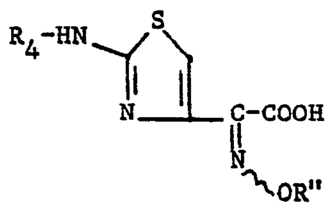

[dans laquelle R" et $R_4$ sont définis comme dans la revendication 6 à l'exception de représenter des atomes d'hydrogène], ou un dérivé réactif de cet acide, sur une amino-7 céphalosporine de formule générale :

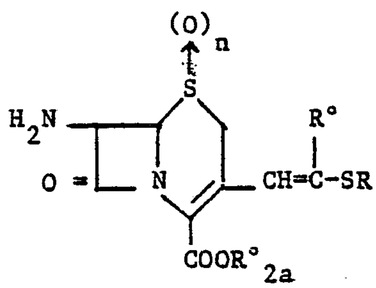

dans laquelle R° et R sont définis comme dans la revendication 1 et $R°_{2a}$ et n sont définis comme dans la revendication 6, puis on réduit l'oxyde obtenu lorsque n = 1, élimine les groupements protecteurs et transforme éventuellement le produit obtenu en un sel d'addition avec un acide, en un sel métallique ou en un sel d'addition avec une base azotée.

8 - Composition pharmaceutique caractérisée en ce qu'elle comprend au moins un produit selon la revendication 1 à l'état pur ou en association avec un ou plusieurs diluants ou adjuvants compatibles et pharmaceutiquement acceptables.

Revendication spéciale Autriche

REVENDICATION

Procédé de préparation d'un nouveau dérivé de la céphalosporine de formule générale :

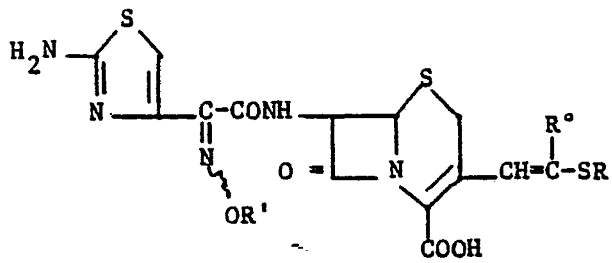

dans laquelle

R est choisi parmi les significations

1) pyridyle-2, -3 ou -4 éventuellement N-oxydés

2) pyrimidinyle-2

3) méthyl-6 oxyde-1 pyridazinyle-3

4) dioxo-5,6 tétrahydro-1,4,5,6 triazine-1,2,4 yle-3 substitué en position -4 par

a) un radical alcoyle contenant 1 ou 2 atomes de carbone éventuellement substitué par un radical alcoyloxy, alcoylthio, formyle,

b) un radical allyle, dihydroxy-2,3 propyle, dihydroxy-1,3 propyle-2 ou formyl-2 hydroxy-2 éthyle,

c) un radical alcoyle contenant 2 ou 3 atomes de carbone substitué par hydroxy, carbamoyloxy, acyloxy ou acylamino (dont les portions acyles sont non substituées ou substituées par amino), alcoylsulfonylamino, uréido, alcoyluréido ou dialcoyluréido,

5) dioxo-5,6 tétrahydro-1,4,5,6 triazine-1,2,4 yle-3 substitué en position -1 ou dioxo-5,6 tétrahydro-1,2,5,6 triazine-1,2,4 yle-3 substitué en position -2 par un radical alcoyle contenant 1 ou 2 atomes de carbone éventuellement substitué par un radical formyle

6) alcoyl-2 dihydro-2,5 oxo-5 triazine-1,2,4 yle-3 éventuellement

substitué en position -6 par un radical alcoyle ou alcoyloxy dont les portions et radicaux alcoyles contiennent 1 ou 2 atomes de carbone,

7) amino-1 dihydro-1,2 oxo-2 pyrimidinyle-4

8) thiadiazol-1,3,4 yle-5 substitué par alcoyle, dialcoylaminoalcoyle ou acylaminoalcoyle,

9) tétrazolyle-5 substitué en position -1 par

a) un radical alcoyle contenant 1 ou 2 atomes de carbone éventuellement substitué par un radical formyle,

b) un radical alcoyle contenant 2 ou 3 atomes de carbone substitué par hydroxy, acylamino, ou dialcoylamino, ou

10) a) alcoyl-1 triazol-1,2,4 yle-5 éventuellement substitué en position -3 par un radical alcoyloxycarbonyle dont les radicaux alcoyle et alcoyloxy contiennent 1 ou 2 atomes de carbone

b) alcoyl-1 triazol-1,3,4 yle-5,

- R° représente un radical d'un premier groupe constitué par un radical phényle éventuellement substitué [par un radical alcoyle, trifluorométhyle, dialcoylaminométhyle, alcoyloxy, alcoylthio, dialcoylamino ou par un atome d'halogène tel que le fluor ou le chlore], un radical hétérocyclyle à 5 ou 6 chaînons contenant 1 seul hétéroatome tel que pyridyle-2 ou -3, thiényle-2 ou -3 ou furyle -2 ou -3 éventuellement substitué par un radical alcoyle, alcoyloxy ou diméthylaminométhyle, ou bien un radical d'un second groupe constitué par un radical alcoyle contenant 1 à 5 atomes de carbone, benzyle éventuellement substitué (par un atome d'halogène ou un radical alcoyle, alcoyloxy, alcoylthio, dialcoylamino ou trifluorométhyle), un radical méthyle substitué par un hétérocycle aromatique à 5 ou 6 chaînons tel que pyridyle-2 ou -3, thiényle-2 ou -3 ou furyle-2 ou -3, un radical cycloalcoyle contenant 3 à 6 chaînons, ou un radical hétérocyclyle à 5 ou 6 chaînons contenant un atome d'oxygène ou de soufre et

R' représente un atome d'hydrogène, un radical alcoyle, vinyle, cyanométhyle ou un radical de formule générale

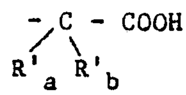

(dans laquelle $R'_a$ et $R'_b$ qui sont identiques ou différents sont des atomes d'hydrogène, des radicaux alcoyle, ou forment ensemble un radical alcoylène contenant 2 ou 3 atomes de carbone), **étant entendu que les substituants R° et -SR peuvent être situés en position E ou Z par rapport au noyau céphalosporine, que le radical -OR' peut se trouver en position syn ou anti et que les radicaux alcoyle et acyle cités ci-dessus contiennent (sauf mention spéciale) 1 à 4 atomes de carbone et sont droits ou ramifiés, ainsi que ses sels d'addition avec les acides, ses sels métalliques et ses sels d'addition avec les bases azotées,** caractérisé en ce que l'on fait agir un thiol (ou un de ses sels alcalins ou alcalinoterreux) de formule générale :

R - SH

[dans laquelle le substituant R, qui est défini comme ci-dessus, est protégé à l'état d'acétal lorsque l'on veut obtenir un produit pour lequel R contient un radical formyle], sur un dérivé de la céphalosporine (ou un mélange des isomères) de formule générale :

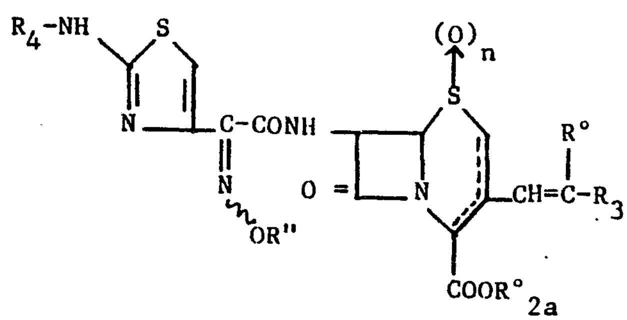

[dans laquelle R° est défini comme ci-dessus,

R" représente un atome d'hydrogène, un radical alcoyle, vinyle, cyanométhyle, un groupement protecteur d'oxime ou un radical de formule générale :

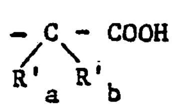

(dans laquelle $R'_a$ et $R'_b$ sont définis comme ci-dessus et dont la fonction acide peut être protégée),et $R_4$ représente un atome d'hydrogène ou un radical protecteur d'amine], $R°_{2a}$ représente un atome d'hydrogène ou un radical protecteur d'acide et le symbole $R_3$ représente un radical de formule générale :

$$R'_3\ SO_2O-$$

dans laquelle $R'_3$ représente un radical alcoyle, trihalogénométhyle, ou phényle éventuellement substitué par un atome d'halogène ou par un ou plusieurs radicaux alcoyle ou nitro, et n égale 0 ou 1, (étant entendu que lorsque n = 0, le produit se présente sous forme bicyclooctène-2 ou -3 et lorsque n = 1, le produit se présente sous forme bicyclooctène-2), puis lorsque n = 1, on réduit le produit obtenu, élimine s'il y a lieu les radicaux protecteurs et transforme éventuellement le produit obtenu en sel d'addition avec un acide, en sel métallique ou en sel d'addition avec une base azotée, ou en ce que l'on fait agir un acide de formule générale :

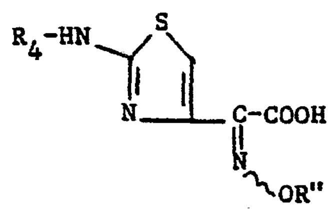

[dans laquelle R" et $R_4$ sont définis comme ci-dessus à l'exception de représenter des atomes d'hydrogène], ou un dérivé réactif de cet acide, sur une amino-7 céphalosporine de formule générale :

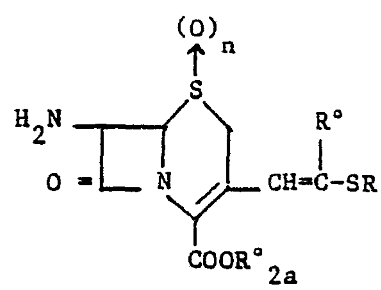

dans laquelle n, $R°_2a$, R° et R sont définis comme ci-dessus, puis on réduit l'oxyde obtenu lorsque n = 1, élimine les groupements protecteurs et transforme éventuellement le produit obtenu en un sel d'addition avec un acide, en un sel métallique ou en un sel d'addition avec une base azotée.

0053542

Office européen des brevets

# RAPPORT DE RECHERCHE EUROPEENNE

Numéro de la demande
EP 81 40 1833

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR - A - 2 081 451 (GLAXO)<br>* Page 1, paragraphes 3,6; page 8, sub. c *<br>-- | 1-8 | C 07 D 501/24<br>A 61 K 31/545 |
| Y | FR - A - 2 137 899 (GLAXO)<br>* Pages 105-116; revendications 1,3,5, sub (i), 6,7, 10b, 21, 22 *<br>---- | 1-8 | |

DOMAINES TECHNIQUES RECHERCHES (Int. Cl. 3)

C 07 D 501/00

CATEGORIE DES DOCUMENTS CITES

X: particulièrement pertinent à lui seul
Y: particulièrement pertinent en combinaison avec un autre document de la même catégorie
A: arrière-plan technologique
O: divulgation non-écrite
P: document intercalaire
T: théorie ou principe à la base de l'invention
E: document de brevet antérieur, mais publié à la date de dépôt ou après cette date
D: cité dans la demande
L: cité pour d'autres raisons

&: membre de la même famille, document correspondant

X Le présent rapport de recherche a été établi pour toutes les revendications

| Lieu de la recherche | Date d'achèvement de la recherche | Examinateur |
|---|---|---|
| La Haye | 04-03-1982 | LUYTEN |

OEB Form 1503.1 06.78